# EUROPEAN PATENT SPECIFICATION

(11) **EP 1 191 026 B1**
(45) Date of publication and mention of the grant of the patent: **19.05.2004**
(21) Application number: 01307528.8
(22) Date of filing: 05.09.2001
(51) Int. Cl.: C07D 403/04, C07D 237/08, A61K 31/505, A61P 9/08

(54) **New pyridazine endothelin antagonists**
Neue Pyridazine als Endothelin-Antagonisten
Nouvelles pyridazines antagonistes d endothéline

(30) Priority: 20.09.2000 GB 0023074
(43) Date of publication of application: 27.03.2002
(73) Proprietor: Pfizer Limited, Sandwich, Kent CT13 9NJ (GB); PFIZER INC., New York, N.Y. 10017 (US)
(72) Inventor: Banks, Bernard Joseph, Sandwich, Kent CT13 9NJ (GB); Chubb, Nathan Anthony Logan, Sandwich, Kent CT13 9NJ (GB); Critcher, Douglas James, Sandwich, Kent CT13 9NJ (GB); Eshelby, James John, Sandwich, Kent CT13 9NJ (GB); Schulz, Darren John, Groton, Connecticut 06340 (GB)
(74) Representative: Atkinson, Jonathan David Mark

(56) References cited:
- EP-A- 0 882 719
- US-A- 4 698 091
- US-A- 5 707 966
- US-A- 5 883 090

## Description

This invention relates to pyridazine derivatives useful in the treatment of a variety of conditions mediated by endothelin and to pharmaceutical formulations containing such compounds useful for the treatment of humans and non-human mammals.

Endothelin (ET) is a potent vasoconstrictor synthesised and released by endothelial cells. There are three distinct isoforms of ET: ET-1, ET-2 and ET-3, all being 21-amino acid peptides and herein the term 'endothelin' refers to any or all of the isoforms. Two receptor subtypes, ET_{A} and ET_{B} have been pharmacologically defined (see for example H. Arai et al., *Nature*, **348,** 730 , 1990) and further subtypes have recently been reported. Stimulation of ET_{A} promotes vasoconstriction and stimulation of ET_{B} receptors causes either vasodilation or vasoconstriction. The main effects of ET are observed in the cardiovascular system, particularly in the coronary, renal, cerebral and mesenteric circulation, and the effects of endothelin are often long-lasting. Stimulation of ET receptors also mediate further biological responses in cardiovascular and non-cardiovascular tissues such as cell proliferation and matrix formation.

Increased circulating levels of endothelin have been observed in patients who have undergone percutaneous transluminal coronary angioplasty (PTCA) (A. Tahara et al., *Metab. Clin. Exp*., 1991, **40,** 1235) and ET-1 has been found to induce neointimal formation in rats after balloon angioplasty (S. Douglas et al., *J. Cardiovasc. Pharm*., 1993, **22** (Suppl 8), 371). The same workers have found that an endothelin antagonist, SB-209670, causes a 50% reduction in neointimal formation relative to control animals (S. Douglas et al., *Circ. Res.,* 1994, 75). Antagonists of the endothelin receptor may thus be useful in preventing restenosis post PTCA. The ET_{A/B} receptor antagonist Bosentan reportedly decreased blood pressure in hypertensive patients (H. Krum et al., New Eng. J. Med., 1998, **338,** 784-790). Antagonists of ET_{B} receptors such as BQ-788 have been demonstrated to increase peripheral resistance in man (*Hypertension*, 1999, **33,** 581-585). Thus ET_{A}-selective receptor antagonists are of benefit in hypertension.

Endothelin-1 is produced in the human prostate gland and endothelin receptors have been identified in this tissue (Y. Saita et al., *Eur. J. Pharmacol*., 1988, **349,** 123-128). Since endothelin is a contractile and proliferative agent, endothelin antagonists are useful in the treatment of benign prostate hypertrophy.

There is widespread localisation of endothelin and its receptors in the central nervous system and cerebrovascular system (R. K. Nikolov et al., *Drugs of Today*, 1992, **28**(5), 303) with ET being implicated in cerebral vasospasm, cerebral infarcts, septic shock, myocardial infarction and neuronal death.

Elevated levels of endothelin have also been observed in patients with:
- recurrent airway obstruction (*Pulm. Pharm. Ther.,* 1998, **11:** 231-235);
- asthma (*Am. J. Resp. Crit. Care Med.,* 1995, **151**:1034-1039);
- acute renal failure (K. Tomita, et al., *Med. Philos*., 1994, **13**(**1**), 64-66);
- chronic renal failure (F. Stockenhuber et al., *Clin. Sci*. (Lond.), 1992, **82,** 255);
- ischaemic Heart Disease (M. Yasuda, *Am. Heart J*., 1990, **119,** 801);
- stable or unstable angina (J. T. Stewart, *Br. Heart J*., 1991, **66,** 7);
- pulmonary hypertension (D. J. Stewart et al., *Ann. Internal Medicine*, 1991, **114**, 464);
- congestive heart failure (R. J. Rodeheffer et al., *Am. J. Hypertension,* 1991, **4,** 9A);
- preeclampsia (B. A. Clark et al., *Am. J. Obstet. Gynecol*., 1992, **166**, 962);
- diabetes (A. Collier et al., *Diabetes Care*, 1992, **15** (8), 1038);
- Crohn's disease (S. H. Murch et al., *Lancet,* 1992, **339,** 381); and
- atherosclerosis (A. Lerman et al., *New Eng. J. Med.,* 1991, **325,** 997).

In every case the disease state associated with the physiologically elevated levels of endothelin is potentially treatable with a substance which decreases the effect of endothelin, such as an endothelin receptor antagonist, or a compound which binds endothelin such that it reduces the effective concentration thereof at the endothelin receptors.

Compounds that antagonise the ET_{A} receptor to a greater extent than the ET_{B} receptor are preferred as ET_{A} receptors are predominantly present in vascular smooth muscles. Blockade of ET_{B} receptor activation may reverse endothelial dependent vasodilation which is beneficial in hypertension. ET may also mediate regeneration of damaged tissue via the ET_{B} receptor, such as proximal tubule cells in the kidney. Thus blockade of ET_{B} receptors, e.g. with a non-selective ET antagonist could inhibit tissue repair. ET_{B} receptors are also involved in the clearance of ET from the systemic circulation. Increased levels of ET are generally considered detrimental. Rises in circulating levels have been observed with non-selective ET antagonists. Treatment with selective ET_{A} receptor antagonists are not likely to induce such rises in circulating levels.

There are a number of publications relating to N-(pyrimidin-4-yl)sulfonamide derivatives having endothelin binding / antagonist activity, for example EP-A-0743307, EP-A-0658548, EP-A-0633259, EP-A-0882719, WO-A-96/20177, EP-A-0801062, WO-A-97/09318, EP-A-0852226, EP-A-0768304, WO-A-96/19459, WO-A-98/03488, EP-A-0601386, EP-A-0510526 and EP-A-0713875.

Various *N*-4-pyrimidinyl sulfonamide derivatives possessing endothelin antagonist activity are described in JP-A-09059160, JP-A-10194972 and JP-A-10226649.

International Patent Application publication number WO-A-96/19455 discloses phenyl and pyridin-4-yl sulfonamides as endothelin antagonists.

We have unexpectedly found that pyridazines of formula (I) below have good affinity for endothelin receptors, and are selective for ET_{A} over ET_{B}. According to the present invention there are provided compounds of formula (I) 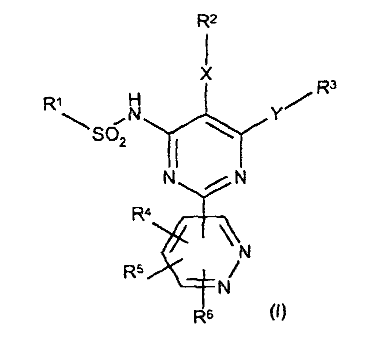 wherein:
R¹ is selected from
   (CR⁷R⁸)ₙ-(C₃₋₈ cycloalkyl), (CR⁷R⁸)ₙ-het¹, (CR⁷R⁸)ₙ-aryl¹,NR⁹R¹⁰ and (CR⁷R⁸)ₙ-het²,
   where R⁷ and R⁸ are independently selected from H and C₁₋₆alkyl, and where
R⁹ and R¹⁰ are independently selected from H or C₁₋₆ alkyl (optionally substituted by one of aryl¹, het¹, het² or (C₃₋₈ cycloalkyl));
n is 2-6;
R² is
   a) Phenyl, optionally fused with het¹ or het²,
   b) naphthyl, optionally fused with het¹ or het² ,or
   c) het²,
   said groups (a), (b) and (c) optionally substituted by C₁₋₆ alkyl, O(C₁₋₆ alkyl), halo, het³, het⁴, aryl², CO₂R¹¹, OC(O)R¹¹, CONR¹¹R¹², S(O)ₚR¹¹ ;
R³ is
   e) C₁₋₆ alkyl,
   f) C₂₋₆ alkenyl,
   g) C₂₋₆ alkynyl,
   h) C₃₋₈ cycloalkyl
Where groups (e), (f), (g) and (h) may be optionally substituted by
OR¹¹, halo, NHC(O)C₁₋₆ alkyl, Ohet¹, Ohet², OC(O)NHhet¹, OC(O)NHhet², NH₂, NHC(O)Ohet², NHC(O)NHhet²;
X is O, S(O)ₚ, NH or a direct link;
Y is O, S(O)ₚ or NH;
p is 0,1 or 2;
R⁴, R⁵ and R⁶ are each independently selected from
H, halo, S(O)ₚR⁹, het¹, het², Aryl¹, O(C₁₋₆ alkyl) and C₁₋₆ alkyl (optionally substituted by halo, OR⁹ or NH₂);
R¹¹ and R¹² are independently selected from H and C₁₋₆ alkyl;
wherein "aryl¹" is a C₆₋₁₄ aromatic carbocycle, including mono-, bi- and tri-cylic systems;
"aryl²" is a C₆₋₁₀ aromatic carbocycle, including mono- and bi-cyclic systems;
"het¹" is a 3-8 membered ring containing 1-3 heteroatoms, each independently selected from N, O and S, said ring being saturated or partially unsaturated and optionally benzofused;
"het²" is a 5 or 6 membered aromatic ring containing 1-3 heteroatoms, each independently selected from N, O and S, said ring being optionally benzofused;
"het³" is a 3-6 membered ring containing 1-3 heteroatoms, each independently selected from N, O and S, said ring being saturated or partially unsaturated and optionally benzofused;
"het⁴" is a 5 or 6 membered aromatic ring containing 1-3 heteroatoms, each independently selected from N, O and S, said ring being optionally benzofused; and wherein "het¹", "het^{2"} and "aryl¹" may optionally be substituted by 1 to 3 substituents independently selected from
   halo, CO₂R⁹, OCOR⁹, OH, het⁴, S(O)ₚR¹¹, O(C₁₋₆alkyl),or C₁₋₆ alkyl (optionally substituted by OH, O(C₁₋₆ alkyl) or halo);
halo includes fluoro, chloro, bromo and iodo groups;
a 6-14 membered aromatic carbocycle includes phenyl, naphthyl, indenyl, anthryl and phenanthryl;
alkyl includes straight chain and branched chain alkyl groups;
and the pharmaceutically acceptable salts, solvates and polymorphs thereof.

A pharmaceutically acceptable salt of a compound of the formula (I) may be readily prepared by mixing together solutions of a compound of the formula (I) and the desired acid or base, as appropriate. The salt may precipitate from solution and be collected by filtration or may be recovered by evaporation of the solvent.

The pharmaceutically acceptable salts of the compounds of the formula (I) include the acid addition and the base salts thereof.

Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, p-toluenesulphonate and pamoate salts.

Suitable base salts are formed from bases which form non-toxic salts and examples are the sodium, potassium, aluminium, calcium, magnesium, zinc and diethanolamine salts.

For a review on suitable salts see Berge *et al., J. Pharm. Sci*., **66**, 1-19, 1977.

The pharmaceutically acceptable solvates of the compounds of the formula (I) include the hydrates thereof.

Also included within the present scope of the compounds of the formula (I) are polymorphs thereof and polymorphs of pharmaceutically acceptable salts and solvates.

It will also be appreciated that the compounds of the invention will include prodrugs thereof: pharmaceutically acceptable derivatives of (I) in which the functional groups explicitly recited above have been derivatised to provide prodrugs which can be converted to the parent compound *in vivo.* Such prodrugs are discussed in Drugs of Today, Vol. 19, 499-538 (1983) and Annual Reports in Medicinal Chemistry, Vol. 10, Ch 31 p306-326.

A compound of the formula (I) may contain one or more asymmetric carbon atoms and therefore exists in two or more stereoisomeric forms. Where a compound of the formula (I) contains an alkenyl or alkenylene group, cis/trans (or E/Z) isomerism may also occur. The present invention includes the individual stereoisomers of the compounds of the formula (I) and, where appropriate, the individual tautomeric forms thereof, together with mixtures thereof.

Racemic substances may either be separated using preparative HPLC and a column with a chiral stationary phase or resolved to yield individual enantiomers utilising methods known to those skilled in the art. In addition, chiral intermediates may be resolved and used to prepare chiral compounds of formulae (I).

Preferred compounds of formula (I) are those where the pyrimidine ring is attached at the 3 or 4 position of the pyridazine ring.

Preferably R¹ is (CR⁷R⁸)ₙ-aryl¹, (CR⁷R⁸)ₙ-het² or (CR⁷R⁸)ₙ-(C₃₋₈ cycloalkyl) and where n is 2, 3, 4, 5 or 6.
More preferably R¹ is (CR⁷R⁸)ₙ-aryl¹ or (CR⁷R⁸)ₙ-(C₃₋₈ cycloalkyl) and where n is 2 or 3.
Most preferably R¹ is (CR⁷R⁸)ₙ-aryl¹ or (CR⁷R⁸)ₙ-(C₃₋₈ cycloalkyl) and where n is 2.

Preferably R² is phenyl or het², both optionally substituted by C₁₋₆ alkyl, O(C₁₋₆ alkyl), halo, het³, het⁴, aryl², CO₂R¹¹, OC(O)R¹¹ or CONR¹¹R¹².
More preferably R² is phenyl optionally substituted by C₁₋₆ alkyl, O(C₁₋₆ alkyl), halo, het³, het⁴, aryl², CO₂R¹¹, OC(O)R¹¹or CONR¹¹R¹².
Most preferably R² is phenyl optionally substituted by C₁₋₃ alkyl, O(C₁₋₃ alkyl), F or Cl.

Preferably R³ is C₁₋₆ alkyl, C₂₋₆ alkynyl or C₃₋₈ cycloalkyl, said alkyl, alkynyl and cycloalkyl groups optionally substituted by
OR¹¹, halo, NHC(O)C₁₋₆ alkyl, Ohet¹, Ohet², OC(O)NHhet¹ or OC(O)NHhet².
More preferably R³ is C₂₋₃ alkynyl or C₁₋₃ alkyl (optionally substituted by
OR¹¹, halo, NHC(O)C₁₋₆ alkyl, Ohet¹, Ohet², OC(O)NHhet¹ or OC(O)NHhet²).
Most preferably R³ is C₁₋₃ alkyl, CH₂CH₂OH, CH₂C≡CH, CH₂CH₂F or CH₂CH₂OCH₃.

Preferably R⁴, R⁵ and R⁶ are selected from H, O(C₁₋₆ alkyl), S(C₁₋₆ alkyl), phenyl, halo, het¹, het² and C₁₋₆ alkyl (optionally substituted by halo or NH₂).
More preferably R⁴ and R⁵ are hydrogen and R⁶ is selected from H, O(C₁₋₆ alkyl) and C₁₋₆ alkyl (optionally substituted by halo).

Most preferably R⁴ and R⁵ are hydrogen and R⁶ is selected from H or C₁₋₆ alkyl.

Preferably R⁷ and R⁸ are independently selected from H and C₁₋₃ alkyl.
More preferably R⁷ and R⁸ are independently selected from H and CH₃.
Most preferably R⁷ and R⁸ are H.

Preferably R⁹ and R¹⁰ are selected from H and C₁₋₃ alkyl (optionally substituted by aryl¹, het¹, het² and C₃₋₈ cycloalkyl).
More preferably R⁹ and R¹⁰ are selected from H and C₁₋₃ alkyl (optionally substituted by aryl¹ or het¹).
Most preferably R⁹ and R¹⁰ are H.

Preferably R¹¹ and R¹² are selected from H and C₁₋₃ alkyl.
More preferably R¹¹ and R¹² are selected from H and CH₃.

Preferably X is O, NH or a direct link.
Most preferably X is O.

Preferably Y is O or NH.
Most preferably Y is O.

Preferably n is 2, 3 or 4.
Most preferably n is 2.

A preferred set of compounds are those described in the Examples and pharmaceutical derivatives thereof.

The invention further provides methods for the production of the compounds of the invention, which are described below and in the Examples and Preparations section. The skilled man will appreciate that the substances of the invention could be made by methods other than those herein described, by adaptation of the methods herein described and/or adaptation of a plethora of methods known in the art. It is to be understood that the synthetic transformation methods specifically mentioned herein may be carried out in various different sequences in order that the desired substances can be efficiently assembled. The skilled chemist will exercise his judgement and skill as to the most efficient sequence of reactions for synthesis of a given target substance.

It will be apparent to those skilled in the art that sensitive functional groups may need to be protected and deprotected during synthesis of a substance of the invention. This may be achieved by conventional techniques, for example as described in 'Protective Groups in Organic Synthesis' by T W Greene and P G M Wuts, John Wiley and Sons Inc, 1991.

Compounds of formula (I) may be prepared in accordance with the following reaction scheme. Unless otherwise specified, the substituents are as defined above with reference to the compounds of formula (I) above.

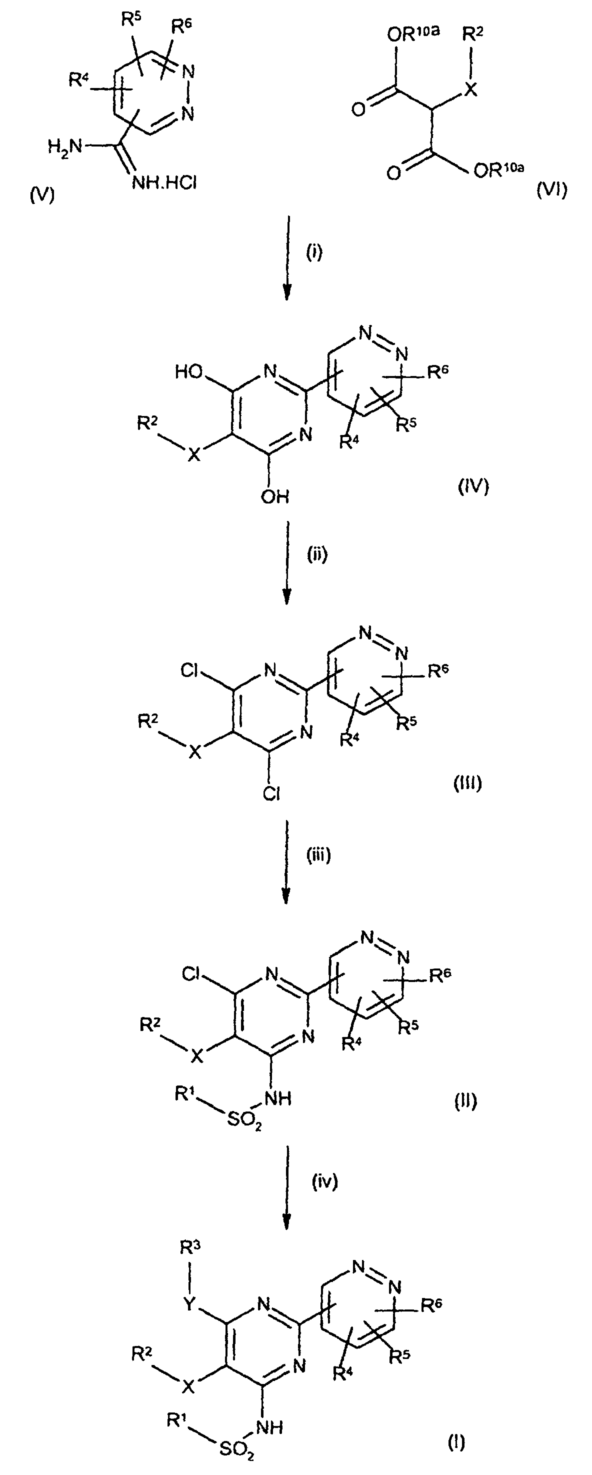

Compounds of formula (IV) may be prepared from compounds of formula (V) and (VI), where R^{10a} is a C₁₋₆ alkyl or phenyl group, by process step (i), a condensation reaction. This is typically conducted in the presence of R^{10a}ONa in a solvent of the corresponding alcohol R^{10a}OH.

Compounds of formula (III) may be prepared by treating compounds of formula (IV) with a chlorinating agent in process step (ii), using, for example, SOCl₂ or phosphorus oxychloride in the presence of a base which may be inorganic eg. sodium hydrogen carbonate or organic eg. diethylisopropylamine. The reaction is optionally conducted employing an inert solvent eg. tetrahydrofuran, optionally cooled or heated, and when tetrahydrofuran is used as solvent, preferably reacted at 60-65°C.

Compounds of formula (II) may be prepared under the conditions of process step (iii), treating compounds of formula (III) with R¹SO₂NH₂ in the presence of a base such as potassium carbonate. The reaction requires heat and should be conducted in a suitable solvent such as DMSO.

R¹SO₂NH₂ may be prepared by treating the corresponding bromide with Na₂SO₃ in water/DME, followed by PCl₅ in eg toluene or POCl₃, followed by reaction with eg aqueous NH₃ in ethanol. R¹Br is commercially available or may be prepared by methods well known to the man skilled in the art.

Compounds of formula (I) may be prepared under the conditions of process step (iv) reacting compounds of formula (II) with R³YH in the presence of a suitable base (eg NaH) and solvent. R³YH is used as a solvent where suitable, with optional use of an inert solvent eg. tetrahydrofuran or dimethylformamide, and optional heat.

Compounds of formula (V) may be prepared in accordance with the following reaction schemes.

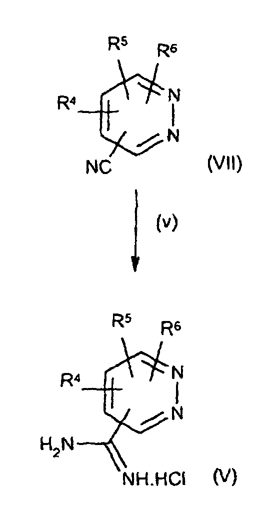

Compounds of formula (V) may be prepared under the conditions of process step (v), treating a compound of formula (VII) with an alkoxide in a solvent of the corresponding alcohol, such as methoxide in methanol, followed by reaction with NH₄Cl. Synthesis of pyridazinecarbonitriles can be found in the literature, such as Dostal *et al*., *Heterocycles* (1986), 24(3), 793-7.

The introduction and transformation of functional groups on aromatic heterocycles is well described in the literature and the principles can be applied to provide the desired pyridazines by one of ordinary skill in the art. Literature references include Tisler *et al*., *Adv*. Heterocycl. *Chem* (1990), 49 385-474 and Heinisch, *Bull. Soc. Chim. Belg*. (1992), 101(7), 579-96.

Compounds of formula (VI) may be prepared in accordance with the following reaction scheme.

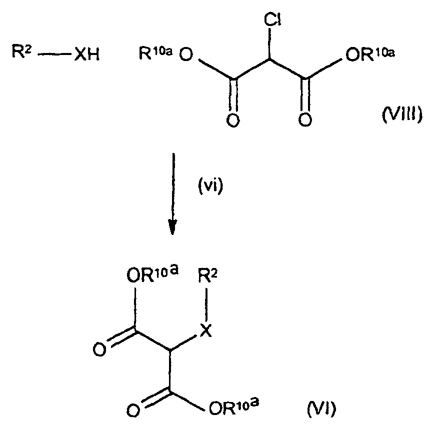

Compounds of formula (VI) may be prepared by reacting a compound of formula (VIII) with R²XH in the presence of an alkoxide or phenoxide in a solvent of the corresponding alcohol (eg. NaOMe in MeOH), or in the case of when R^{10a} is phenyl, a suitable inert solvent, eg, tetrahydrofuran. Compounds of formula (VIII) and R²XH are commercially available, or may be easily prepared by a man skilled in the art. Where X is a direct link, compounds of formula (VI) can be made using literature methods such as those found in US patent 5,750,766.

Compounds of formula (IA) where R¹, R², R⁴, R⁵, R⁶ and X are as described above and the YR³ chain is OCH₂CH₂OH, with the proviso that R⁴, R⁵ and R⁶ are not CO₂R⁹ or alkyl chloride or bromide, may be further elaborated to give the corresponding compound of formula (IB) where YR³ is OCH₂CH₂OC(O)NHhet². These compounds may be prepared in accordance with the following reaction scheme.

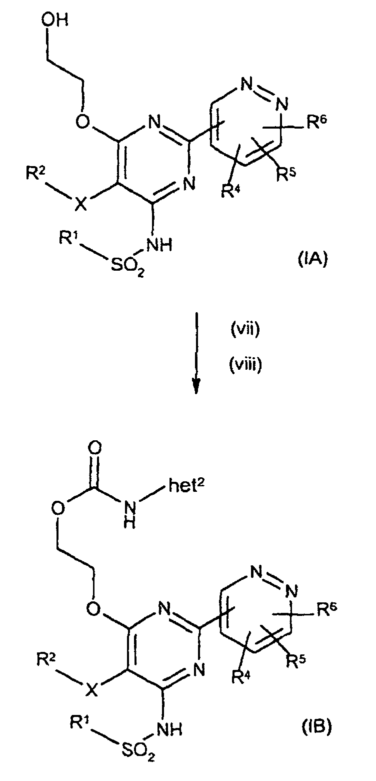

Compounds of formula (IA) are reacted under the conditions of process step (vii) with an agent to activate the alcohol such as phosgene or carbonyldiimidazole. This intermediate is then reacted under the conditions of process step (viii) with H₂N-het² in the presence of a suitable base such as triethylamine to give (IB).
Compounds of formula (IA) where R¹, R², R⁴, R⁵, R⁶ and X are as described above and the YR³ chain is OCH₂CH₂OH, with the proviso that R⁴, R⁵ and R⁶ are not CO₂R⁹ or alkyl chloride or bromide, may be further elaborated to give the corresponding compound of formula (IC) where YR³ is OCH₂CH₂Ohet². These compounds may be prepared in accordance with the following reaction scheme.

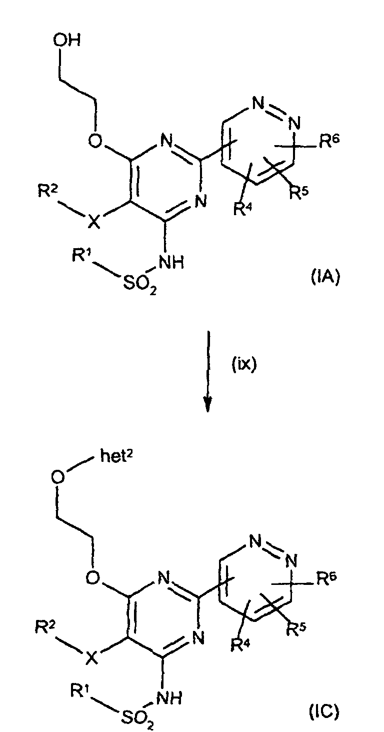

Compounds of formula (IA) were treated under the conditions of process step (ix) which comprised reaction with het²-Z, where Z is a suitable leaving group such as halo or SO₂Me, in the presence of a suitable base such as sodium hydride.

Compounds of formula (ID) where R¹, R², R³, R⁴, R⁵, Y and X are as described above and R⁶ is CO₂R⁹, with the proviso that there are no other ester, ketone or hydroxy moieties within the molecule, may be further elaborated to give the corresponding compound of formula (IF) where R⁶ is CH₂F or (IG) where R⁶ is CF₂H. These compounds may be prepared in accordance with the following reaction scheme.

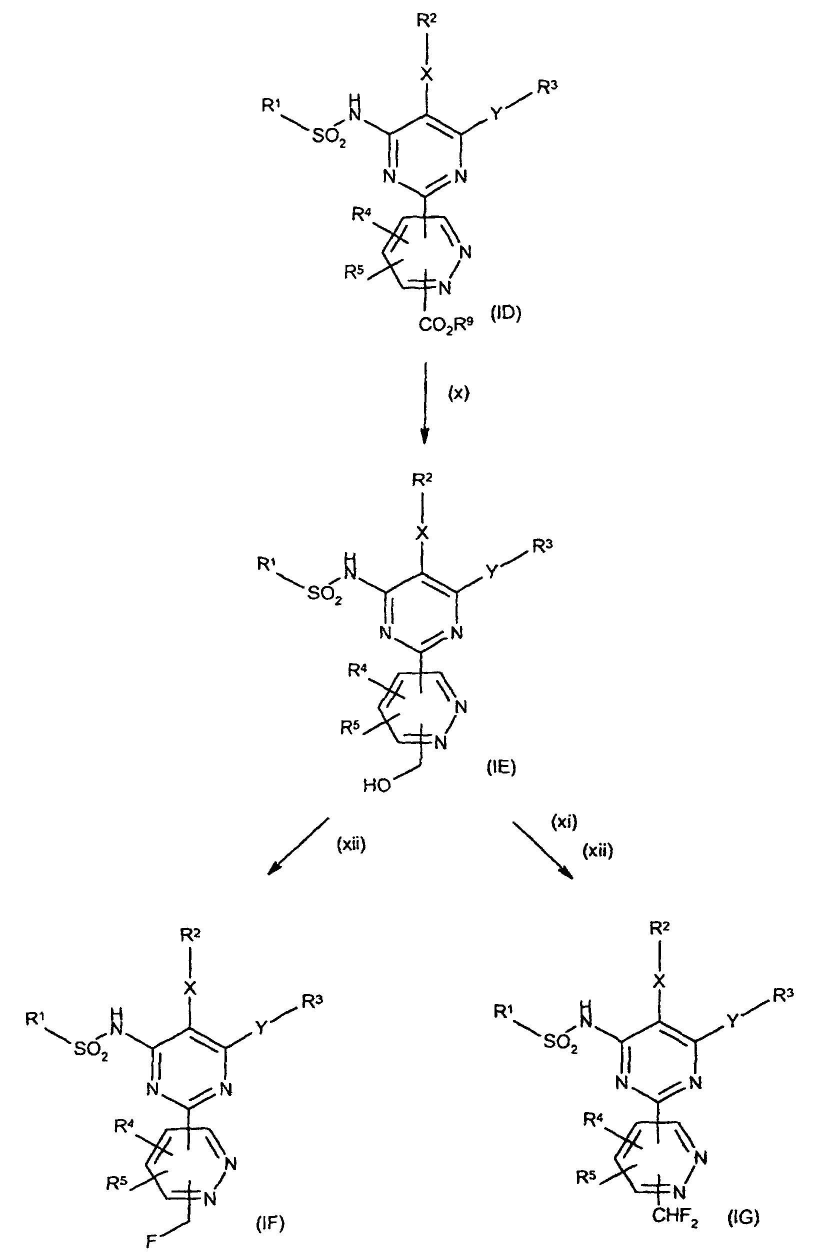

Compounds of formula (ID) are treated under the conditions of process step (x) a reduction, with a suitable reagent such as DIBAL or LiAlH₄ to give the corresponding alcohol (IE). The alcohol may be oxidised to an aldehyde via process step (xi) using a suitable oxidant such as pyridinium dichromate or MnO₂, followed by fluorination in process step (xii) by reaction with a suitable fluorinating agent such as diethylaminosulfur trifluoride (DAST) to give compound (IG). Compounds of formula (IE) may also be converted to compounds of formula (IF) under the conditions of process step (xii) described above.

The compounds of the invention may be separated and purified by conventional methods.

The substances of the invention are useful because they blockade ET receptors and are thus useful in the treatment or prevention of any diseases for which such a blockade is beneficial. More particularly, they are useful in the treatment and prevention of restenosis, acute/chronic renal failure, hypertension including pulmonary and systemic hypertension; benign prostatic hypertrophy, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn. The treatment of congestive heart failure, restenosis, renal failure and systemic and pulmonary hypertension are of particular interest. The substances of the invention may be administered alone or as part of a combination therapy.

The invention also provides a compound of formula (I) or a pharmaceutically acceptable derivative thereof for use as medicament.

The invention also provides for the use of a compound of formula (I) or pharmaceutically acceptable derivative thereof as defined above, in the manufacture of a medicament for the treatment of conditions mediated by endothelin, particularly ET_{A}, more particularly restenosis, acute/chronic renal failure, pulmonary hypertension, systemic hypertension; benign prostatic hyperplasia, male erectile dysfunction, prostate cancer, metastatic bone cancer, congestive heart failure, stroke, subarachnoid haemorrhage, angina, atherosclerosis, cerebral and cardiac ischaemia, prevention of ischaemia/reperfusion injury (e.g. allografts), cyclosporin induced nephrotoxicity, glaucoma, radiocontrast nephropathy, diabetic neuropathy, allergy, restoration of organ perfusion in haemorrhagic shock, lipoprotein lipase related disorders, chronic obstructive pulmonary disease and hyaline membrane disease in newborn.

The invention also provides a method of treating conditions mediated by endothelin, particularly ET_{A}, which comprises administering to a patient in need of such treatment a therapeutically effective amount of a compound of formula (I) or a pharmaceutically acceptable derivative thereof.

Reference to treatment herein includes prevention of undesirable conditions as well as alleviation or cure of said conditions.

The biological activity of the substances of the invention may be demonstrated as follows:

### Human Binding assay

Competition between test substances and ¹²⁵I-ET-1 binding to human endothelin receptors is determined as follows.

### Binding to ET_{A} receptors

25µl of a 30pM solution of [¹²⁵I]Tyr¹³ ET-1 (specific activity 2,200Ci/mM) is mixed with 25µl samples of test substance (final concentrations in the range 0.1 nM - 50,000nM). 200µl of a solution containing cloned human ET_{A} receptor (0.75pmoles receptor protein/ml), 50mM Tris, 0.5mM CaCl₂, 0.1% human serum albumen, 0.1% bacitracin, 0.05% Tween 20, pH 7.4 is added. The solution is mixed at 37°C for 2 hours. After the incubation, the unbound ligand is separated from receptor bound ligand by filtration with a Brandel cell harvester, followed by three washes of buffer. Filter papers are counted for radioactivity, and the IC₅₀ (the concentration of test compound at which 50% of the radiolabelled compound is unbound) determined for the concentration range tested.

### Binding to ET_{B} receptors

25µl of a 30pM solution of [¹²⁵I]Tyr¹³ ET-1 (specific activity 2,200Ci/mM) is mixed with 25µl samples of test substance (final concentration 0.1 nM - 50,000nM). 200µl of a solution containing cloned human ET_{B} receptor (0.25pmoles receptor protein/ml), 50mM Tris, 0.5mM CaCl₂, 0.1% human serum albumen, 0.1% bacitracin, 0.05% Tween 20, pH 7.4 is added. The solution is mixed at 37°C for 2 hours. After the incubation, the unbound ligand is separated from receptor bound ligand by filtration with a Brandel cell harvester, followed by three washes of buffer. Filter papers are counted for radio-activity, and the IC₅₀ (the concentration of test compound at which 50% of the radiolabelled compound is unbound) determined for the concentration range tested.

The compounds of the present invention were tested and found to have good affinity for endothelin receptors and to be selective for ET_{A} over ET_{B}.

### Dog Binding assay

Competition between test substances and ligands binding to canine endothelin receptors is determined as follows:

### Dog ET_{A} Binding Assay

50µl of a 500pM solution of ¹²⁵I-PD-151242 (Specific activity 2,000 Ci/mM) is mixed with 50µl samples of test substances (final concentrations in the range from 0.01-10,000nM). 100µg of purified dog kidney homogenate is added in 150µl of the following buffer: 50mM Tris, 10mM MgCl₂ and 0.05% Bovine Serum Albumen at pH 7.4. The solution is incubated at room temperature for 2 hours. After the incubation, the unbound ligand is separated from receptor bound ligand by filtration with a Brandel cell harvester, followed by 5 washes with buffer (Tris 50mM, MgCl₂ 10mM). Filter papers are counted for radioactivity and the Kᵢ (an IC₅₀ corrected for the dissociation constant and concentration of the ligand added) is determined for the concentration range tested.

### Dog ET_{B} Binding Assay

50µl of a 100pM solution of ¹²⁵I-IRL-1620 (Specific activity 2,200 Ci/mM) is mixed with 50µl samples of test substances (final concentrations in the range from 0.01-10,000nM). 50µg of purified Dog cerebellum homogenate is added in 150µl of the following buffer; 50mM Tris, 10mM MgCl₂ and 0.05% Bovine Serum Albumen at pH 7.4. The solution is incubated at 30°C for 90 minutes. After the incubation, the unbound ligand is separated from receptor bound ligand by filtration with a Brandel cell harvester, followed by 5 washes with buffer (Tris 50mM, MgCl₂ 10mM). Filter papers are counted for radioactivity and the Kᵢ (an IC₅₀ corrected for the dissociation constant and concentration of the ligand added) is determined for the concentration range tested.

The compounds of the present invention were investigated using the above assay and demonstrated strong ET_{A} affinity and a marked selectivity for the ET_{A} over the ET_{B} receptor. Of the compounds disclosed, the compound of example 5 displays affinity and selectivity for the dog ET_{A} receptor (Ki 2nM) vs dog ET_{B} (Ki 132nM).

According to a further aspect of the invention, there are provided pharmaceutical formulations comprising a substance of the invention, as defined above, and a pharmaceutically-acceptable adjuvant, diluent or carrier.

The substances of the invention can be administered alone but will generally be administered in admixture with a pharmaceutical carrier selected with regard to the intended route of administration and standard pharmaceutical or veterinary practice. For example they can be administered orally in the form of tablets containing such excipients as starch or lactose or in capsules or ovules either alone or in admixture with excipients or in the form of elixirs, solutions or suspensions containing the substance in a liquid carrier, for example a vegetable oil, glycerine or water with a flavouring or colouring agent. They can be injected parenterally, for example intravenously, intramuscularly or subcutaneously. For parental administration, they are best used as sterile aqueous solutions which may contain other substances, for example, enough glucose or salts to make the solution isotonic with blood. For parenteral administration the substance may also be administered as a solution or suspension in a suitable oil, for example polyethylene glycol, lecithin or sesame oil.

Substances of the invention may also be administered through inhalation of a solution, suspension or emulsion that may be administered as a dry powder or in the form of an aerosol using a conventional propellant such as dichlorodifluoromethane.

For oral or parenteral administration to human patients the daily dosage levels of substances of the invention will be from 0.01 to 30 mg/kg (in single or divided doses) and preferably will be in the range 0.01 to 5 mg/kg. Thus tablets will contain 1mg to 0.4g of substance for administration singly or two or more at a time, as appropriate. The above dosages are, of course only exemplary of the average case and there may be instances where higher or lower doses are merited, and such are within the scope of the invention.

### General Example

A formulation of the tablet could typically contain between about 0.01 mg and 500mg of active compound whilst tablet fill weights may range from 50mg to 1000mg. An example of a formulation for a 10mg tablet is illustrated below:

| Ingredient | %w/w |
|---|---|
| Free acid, Free base or Salt form of active compound | 10.000 |
| Lactose | 64.125 |
| Starch | 21.375 |
| Croscarmellose sodium | 3.000 |
| Magnesium Stearate | 1.500 |

The tablets are manufactured by a standard process, for example, direct compression or a wet or dry granulation process. The tablet cores may be coated with appropriate overcoats.

Alternatively the substances of the invention can be administered in the form of a suppository or pessary, or they may be applied topically in the form of a lotion, solution, cream, ointment or dusting powder or in the form of a medicated plaster, patch or membrane. For example they may be incorporated in a cream containing an aqueous emulsion of polyethylene glycols or liquid paraffin. The compounds may also be administered intranasally.

For veterinary use although it is possible to administer a substance of the invention directly without any formulation, the substances are preferably employed in the form of a pharmaceutical or veterinary formulation comprising a pharmaceutically or veterinarily acceptable carrier, diluent or excipient and a substance of the invention. Such compositions will contain from 0.1 percent by weight to 90.0 percent by weight of the active ingredient.

The methods by which the compounds may be administered include oral administration by capsule, bolus, tablet or drench, topical administration as an ointment, a pour-on, spot-on, dip, spray, mousse, shampoo or powder formulation or, alternatively, they can be administered by injection (e.g. subcutaneously, intramuscularly or intravenously), or as an implant.

Such formulations are prepared in a conventional manner in accordance with standard veterinary practice. Thus capsules, boluses or tablets may be prepared by mixing the active ingredient with a suitable finely divided diluent or carrier additionally containing a disintegrating agent and/or binder such as starch, lactose, talc or magnesium stearate, etc. Oral drenches are prepared by dissolving or suspending the active ingredient in a suitable medium. Pouron or spot-on formulations may be prepared by dissolving the active ingredient in an acceptable liquid carrier vehicle such as butyl digol, liquid paraffin or a non-volatile ester, optionally with the addition of a volatile component such as propan-2-ol.

Alternatively, pour-on, spot-on or spray formulations can be prepared by encapsulation, to leave a residue of active agent on the surface of the animal. Injectable formulations may be prepared in the form of a sterile solution which may contain other substances, for example enough salts or glucose to make the solution isotonic with blood. Acceptable liquid carriers include vegetable oils such as sesame oil, glycerides such as triacetin, esters such as benzyl benzoate, isopropyl myristrate and fatty acid derivatives of propylene glycol, as well as organic solvents such as pyrrolidin-2-one and glycerol formal. The formulations are prepared by dissolving or suspending the active ingredient in the liquid carrier such that the final formulation contains from 0.1 to 10% by weight of the active ingredient.

These formulations will vary with regard to the weight of active substance contained therein, depending on the species of animal to be treated, the severity and type of infection and the body weight of the animal. For parenteral, topical and oral administration, typical dose ranges of the active ingredient are 0.01 to 100 mg per kg of body weight of the animal. Preferably the range is 0.1 to 10 mg per kg.

The compositions are preferably formulated in a unit dosage form, each dosage containing from about 1 to about 500 mg, more usually about 5 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

As an alternative for veterinary use the substances may be administered with animal feedstuff and for this purpose a concentrated feed additive or premix may be prepared for mixing with the normal animal feed.

The compounds of the formula (I) may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

### EXAMPLES AND PREPARATIONS

High performance liquid chromatography (HPLC) retention times and UV spectra were recorded using a Hewlett-Packard 1090 LUSI diode-array spectrophotometer (method A). All NMR spectra were measured in CDCl₃ or MeOD by an Inova 300 MHz or 400 MHz spectrometer unless otherwise indicated and peak positions are expressed in parts per million (ppm) downfield from tetramethylsilane. The peak shapes are denoted as follows: s, singlet; d, doublet; t, triplet; q, quartet; m, multiplet; br broad. High resolution MS data was acquired on an AutoSpecQ with electrospray ionisation (ESI) or thermospray ionisation (TSPI) using a PEG reference (or on a Bruker Apex II FTMS with ESI where indicated). All calculated MS values for compounds including Cl are based on the ³⁵Cl isomer. All IR spectra were recorded using a Perkin Elmer Paragon 1000 FT-IR.

### PREPARATION 1

### 3-Pyridazinecarboximidamide hydrochloride

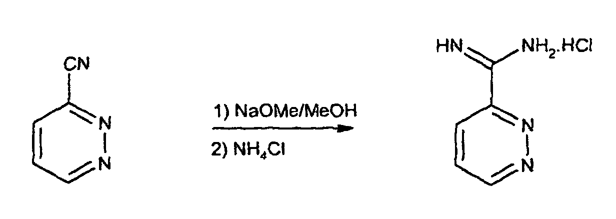

Sodium methoxide (25% w/v in methanol, 0.3ml, 1.3 mmol) was added in one portion to a stirred solution of 3-cyanopyridazine (2.4g g, 22.9 mmol) (Ref. *Heterocycles*, **1986,** 24(3), 793) in methanol (10ml). The resulting mixture was stirred at room temperature for 16 hours. Ammonium chloride (2.45g, 45.8 mmol) was added to the reaction mixture, which was then stirred for 30 minutes before being ultrasonicated for a further 15 minutes. A fine, yellow precipitate was observed after this time. The solvent was evaporated under vacuum and the residue was suspended in ethanol (150ml) and heated to reflux for 5 minutes. The hot mixture was filtered (gravity) to remove unreacted ammonium chloride and sodium chloride and was then cooled to room temperature. A pale yellow, crystalline solid formed upon cooling and this was isolated by suction filtration to give the title compound (2.03g), m.p. 234 - 241°C.
¹H-NMR (300 MHz, DMSO-D₆): δ 8.08 (dd, 1H), 8.54 (d, 1H), 9.53 (d, 1H) ppm. IR (KBr disk): λ 1404, 1692, 3040, 3143, 3431, 3469 cm⁻¹.

### Preparation 2

### 5-(2-Methoxyphenoxy)-2-(3-pyridazinyl)-4,6-pyrimidinediol

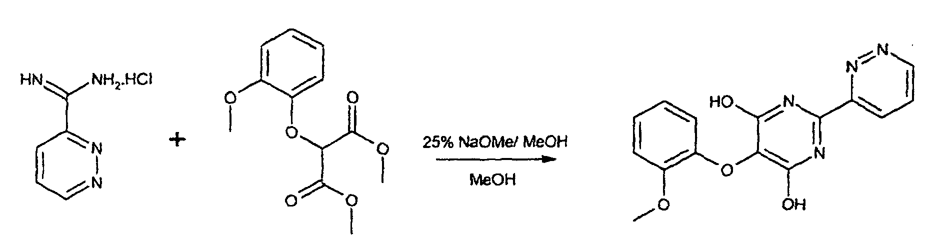

Dimethyl 2-(2-methoxyphenoxy)malonate (3.05g, 12.6mmol) (Ref. Canadian Patent Application CA2071193A, 1992) in methanol (15ml) was added over 5 minutes to a stirred, 25% w/v solution of sodium methoxide in methanol (8.58ml, 37.8mmol of sodium methoxide) at room temperature. The resulting mixture was stirred for 10 minutes and then a solution of 3-pyridazinecarboximidamide hydrochloride (2.0g, 12.6mmol) in methanol (15ml) was added dropwise over 5 minutes and the resulting mixture was stirred at room temperature overnight. The reaction mixture was concentrated under vacuum and then diluted with water (100ml). The aqueous solution was acidified to pH 4 with sulfuric acid (20%), resulting in the formation of the title compound as a purple solid, which was isolated by filtration (1.4g).
¹H-NMR (300 MHz, DMSO-d₆): δ 3.83 (s, 3H), 6.68 (d, 1H), 6.78 (t, 1H), 6.93 (t, 1 H), 7.03 (d, 1 H), 7.94 (dd, 1H), 8.34 (d, 1 H), 9.43 (d, 1 H) ppm.
HRMS (+ve ion) found: m/z 313.0929 (MH⁺). C₁₅H₁₂N₄O₄+H requires m/z 313.0932

### PREPARATION 3

### 3-[4,6-Dichloro-5-(2-methoxyphenoxy)-2-pyrimidinyl]pyridazine

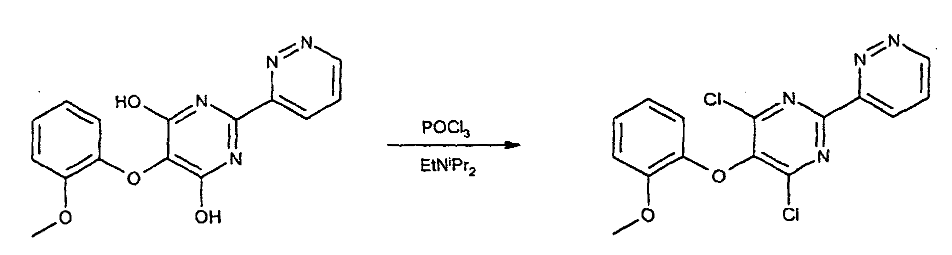

A solution of 5-(2-methoxyphenoxy)-2-(3-pyridazinyl)-4,6-pyrimidinediol (1.4g, 4.5mmol) and diisopropylethylamine (1.28g, 9.9mmol) in phosphorus oxychloride (7ml) was stirred for 1 hour at room temperature under a nitrogen atmosphere and then heated under reflux for 1 hour before being cooled to room temperature and stirred for 72 hr. The reaction mixture was heated under reflux for a further 7 hours and then again cooled to room temperature and stirred for a further 16 hours.
The reaction mixture was added slowly to a stirred aqueous solution of hydrochloric acid (0.1M, 200ml) at 50°C over 20 minutes. A precipitate formed and was isolated by filtration. The isolated crude product was purified by chromatography on a Biotage® Flash 40(s) silica cartridge (40g) using 70% ethyl acetate/ heptane as eluant to give the title compound as a light brown solid (1.1g), R_{f} 0.59 (ethyl acetate).
¹H-NMR (300 MHz, CDCl₃): δ 3.86 (s, 3H), 6.79 (d, 1 H), 6.91 (t, 1H), 7.02 (d, 1H), 7.13 (t, 1H), 7.72 (dd, 1 H), 8.58 (dd, 1H), 9.36 (d, 1H) ppm.
HRMS (+ve ion) found: m/z 349.0247 (MH⁺). C₁₅H₁₀³⁵Cl₂N₄O₂+H requires m/z 349.0253.

### PREPARATION 4

### 2-(4-Fluorophenyl)ethanesulfonic acid sodium salt

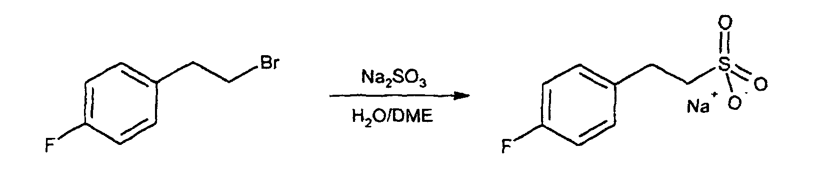

A mixture of 2-(4-fluorophenyl)ethyl bromide (1.8g, 8.9mmol) (Ref. *Chim. Ther*., 1969, **4**(3), 185) and sodium sulfite (2.24g, 8.9mmol) in water (5ml) and dimethoxyethane (5ml) was heated under reflux for 21 hours. The mixture was then left for 72 hours at room temperature. The mixture was concentrated under vacuum to leave the title compound as a crytalline mixture with sodium bromide (2.87g).
¹H-NMR (300 MHz, D₂O): δ 2.96 (m, 2H), 3.09 (m, 2H), 7.03 (dd, 2H), 7.25 (dd, 2H) ppm.
LRMS (-ve ion) found: m/z 203 (M⁻). C₈H₈FO₂S requires 203.

The following examples were also prepared by a similar method to that described for preparation 4.

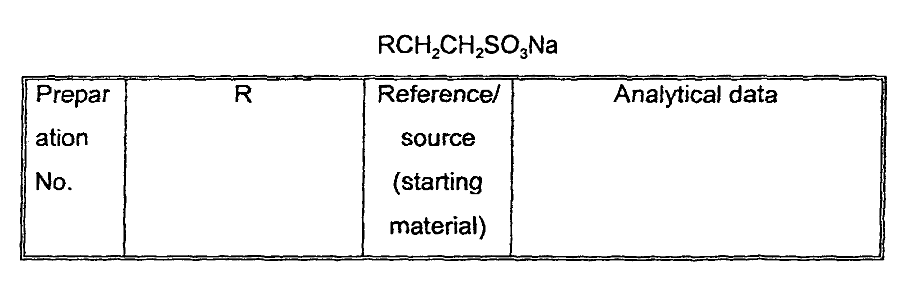

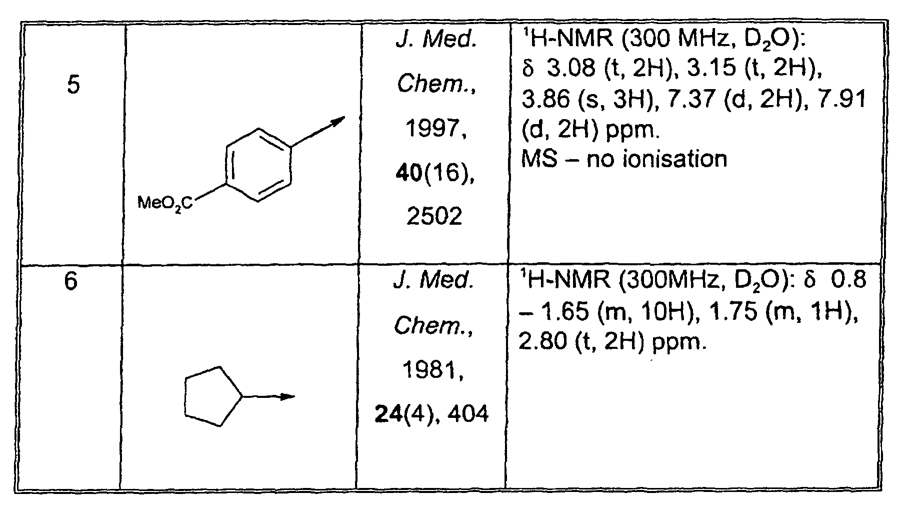

### PREPARATION 7

### 2-(4-Fluorophenyl)ethanesulfonyl chloride

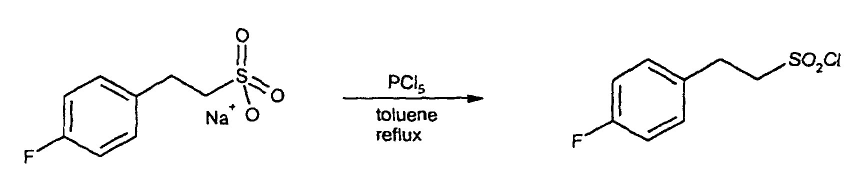

Phosphorus pentachloride (5.6g, 27mmol) was added portionwise over 10 minutes to a stirred suspension of 2-(4-fluorophenyl)ethanesulfonic acid sodium salt ( 9mmol, contaminated with sodium bromide, total weight 2.87g) in dry toluene (15ml) at room temperature. The reaction mixture was heated under reflux for 90 minutes and was then stirred at room temperature overnight. The reaction mixture was diluted with water (20ml) and the organic phase was separated. The aqueous phase was extracted with dichloromethane (20ml) and the organic extracts were combined, dried over magnesium sulfate and concentrated under vacuum to give the title compound as an oil, (0.7g) R_{f} 0.92 (dichloromethane).
¹H-NMR (300 MHz, CDCl₃): δ 3.33 (m, 2H), 3.89 (m, 2H), 7.07 (dd, 2H), 7.23 (dd, 2H) ppm.
IR (NaCI disk): λ 1599, 1509, 1450, 1354 cm⁻¹

The following compounds were prepared by similar methodology.

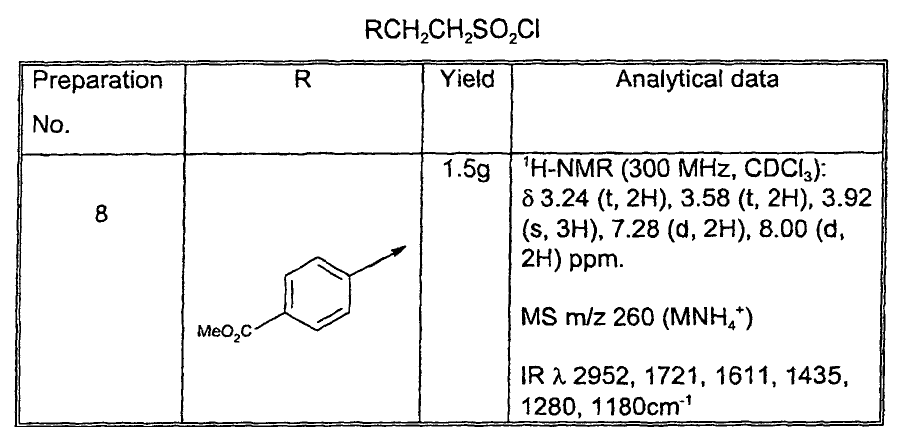

### PREPARATION 9

### 2-(Cyclopentyl)ethylsulfonyl chloride

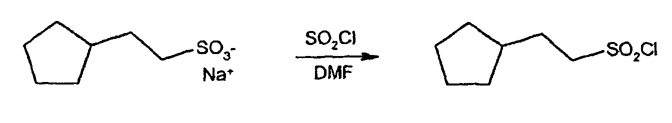

Dimethylformamide (5 drops) was added over 1 minute to a stirred solution of 2-(cyclopentyl)ethylsulfonic acid sodium salt (15.5g, 87.5mmol) in thionyl chloride (60ml) under a nitrogen atmosphere. The reaction mixture was stirred under reflux for 5 hours and was then cooled to room temperature and concentrated under vacuum. The residue was suspended in toluene (100ml) and then concentrated under vacuum. The residue was dissolved in a mixture of ethyl acetate (150ml) and water (150ml) and the organic phase was separated. The aqueous phase was extracted with ethyl acetate (100ml) and the combined organic extracts were washed with brine (3 x 100ml), dried (magnesium sulfate) and evaporated to give the title compound as a brown oil (14.1g), R_{f} 0.5 (pentane/ethyl acetate 95:5).
¹H-NMR (300 MHz, CDCl₃): δ 1.15 (m, 2H), 1.50 -1.72 (m, 4H), 1.84 (m, 2H), 1.91 (m, 1H), 2.03 (m, 2H), 3.66 (m, 2H) ppm.

### PREPARATION 10

### 2-(4-Fluorophenyl)ethanesulfonamide

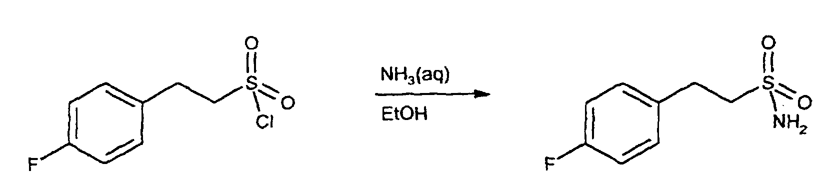

A mixture of 2-(4-flourophenyl)ethanesulfonyl chloride (0.7g, 3.3mmol) and concentrated aqueous ammonia (0.88 specific gravity, 10ml) in ethanol (5ml) was stirred at room temperature overnight. The reaction mixture was diluted with water (10ml), acidified to pH 4 with citric acid (1.0M) and then extracted with dichloromethane. The organic extracts were dried by filtering through an organic-only, permeable membrane and then concentrated under vacuum. The residue was purified by chromatography on a Biotage® Flash 40 (s) column (40g silica) using 10 - 20% ethyl acetate/dichloromethane as eluant to give the title compound as an off white solid, (0.66g).
¹H-NMR (300 MHz, CDCl₃): δ 2.98 (m, 2H), 3.23 (m, 2H), 7.11 (dd, 2H), 7.29 (dd, 2H) ppm.
IR (KBr disk): λ 3357, 3254, 3068, 2962, 1907, 1598, 1509, 1321, 1161 cm⁻¹ LRMS (-ve ion) found: m/z 202 (M-H). C₈H₁₀FNO₂S - H requires m/z 202

The following compounds were prepared by similar methodology.

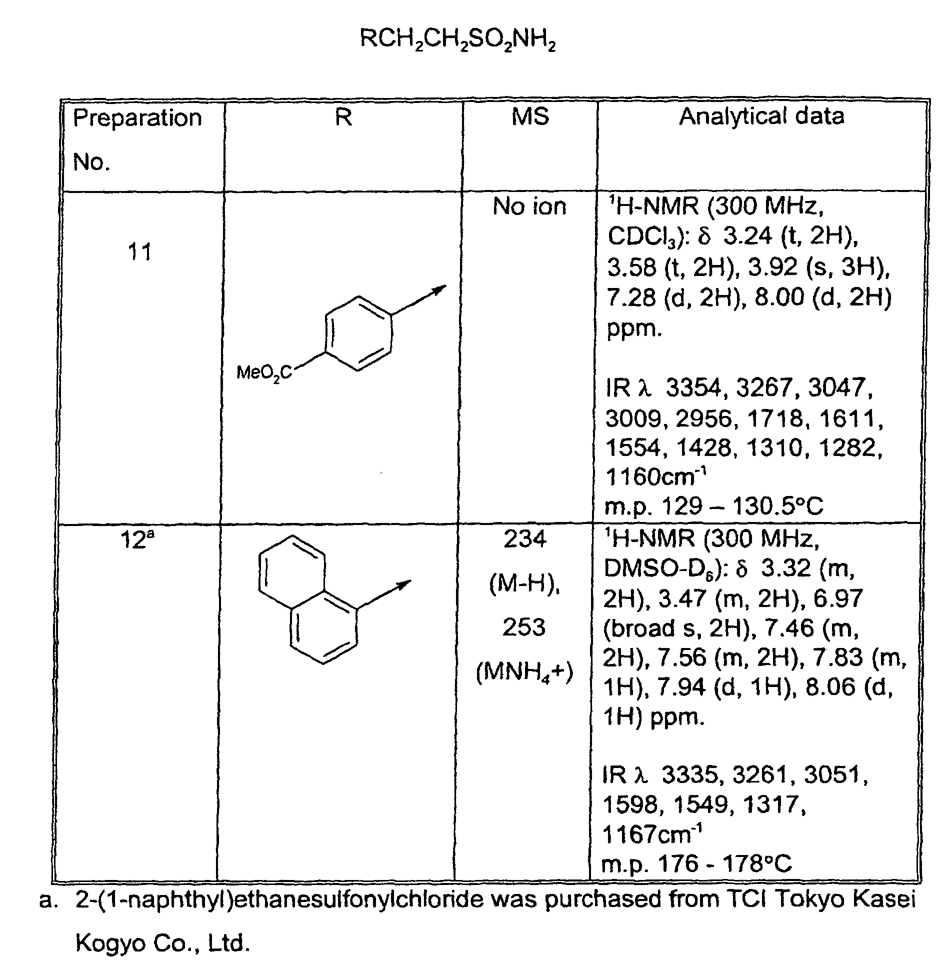

### PREPARATION 13

### 2-(Cyclopentyl)ethanesulfonamide

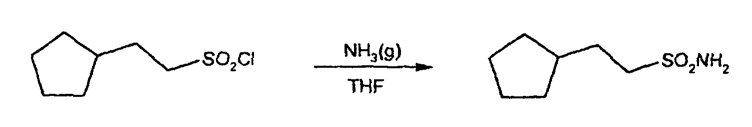

A solution of 2-(cyclopentyl)ethanesulfonyl chloride (14.1g, 71.4mmol) in dry tetrahydrofuran (40ml) was saturated with gaseous ammonia at 0°C. The resulting solution was stirred at room temperature under nitrogen for 2 hours and then concentrated under vacuum. The residue was dissolved in ethyl acetate (50ml) and water (50ml) and the aqueous phase was then acidified to pH 2 with 2.0M aqueous hydrochloric acid. The organic phase was separated and then washed with water (2 x 30ml), dried (sodium sulfate) and concentrated under vacuum. Purification of the residue by chromatography on silica (500g) using 65/35 pentane/ethyl acetate as eluant gave the title compound as an off-white solid (6.48g), R_{*f*} 0.7 (pentane/ethyl acetate 1:1).
¹H-NMR (300 MHz, CDCl₃) δ 1.12 (m, 2H), 1.48 -1.68 (m, 4H), 1.73 - 1.94 (5H, m), 3.10 (m, 2H), 4.55 (broad s, 2H) ppm
LRMS (+ve ion) found: m/z 195 (MNH₄⁺). C₇H₁₅NO₂S + NH₄⁺ requires m/z 195

### PREPARATION 14

### N-[6-Chloro-5-(2-methoxyphenoxy)-2-(3-pyridazinyl)-4-pyrimidinyl]-2-(1-naphthyl)ethanesulfonamide

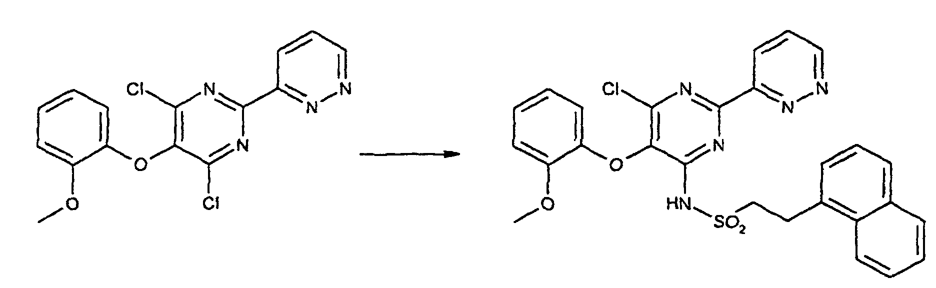

To a solution of 3-[ 4,6-dichloro-5-(2-methoxyphenoxy)-2-pyrimidinyl] pyridazine, (0.06g, 0.17mmol) in dimethylsulfoxide (4ml) was added potassium carbonate (0.13g, 0.94mmol) immediately followed by 2-(1-naphthyl)ethanesulfonamide (0.044g, 0.18mmol). The mixture was stirred vigorously for 72 hours at 90°C and then quenched with hydrochloric acid solution (2M, 4ml). Ice-cold water (5ml) was added and a white precipitate was observed. The resultant mixture was partitioned between water (10ml) and dichloromethane (3x10ml). The combined organic extracts were washed with water (3x10ml), dried (magnesium sulfate) and concentrated under vacuum, yielding a yellow oil. Trituration with 1 ml of ice cold water, followed by filtration gave the title compound as a white solid (0.088g), m.p. 191.5 °C.
¹H-NMR (300 MHz, CDCl₃) δ 3.6 (t, 2H), 3.98 (s, 3H), 4.12 (t,2H), 6.91 (t, 1H), 7.02 (d, 1H), 7.1-7.3 (m, 7H), 7.7(d,1H), 7.8 (t, 1H), 8.3 (d, 1H), 9.3 (d, 1H) ppm.
IR (NaCl disk) λ 2996.5, 2913.0, 1436.9, 1406.2, 1057.2 cm⁻¹.
LRMS (+ve ion) found: m/z 549 (MH⁺). C₂₇H₂₂³⁵ClN₅O₄S+H requires m/z 549.

The compounds of the following tabulated preparations of the general formula: 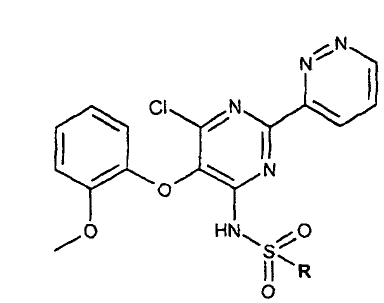 were prepared by a similar method to that of Preparation 14 using the appropriate sulfonamide starting materials and the dichloropyrimidine of Preparation 3.

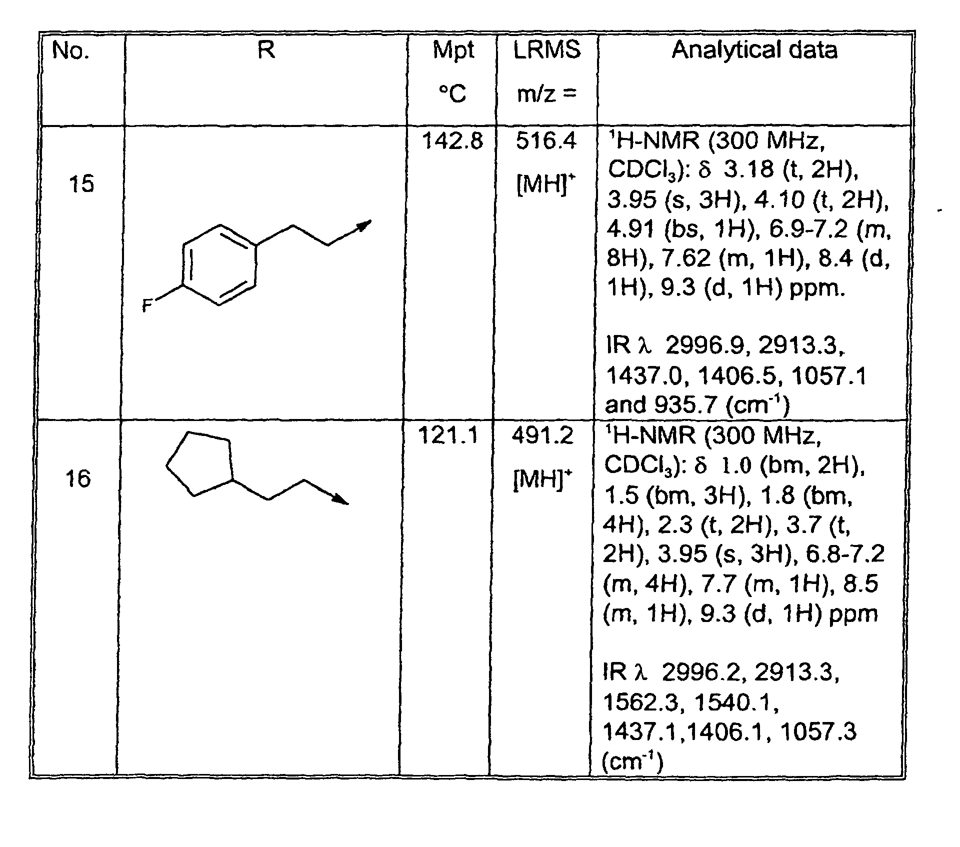

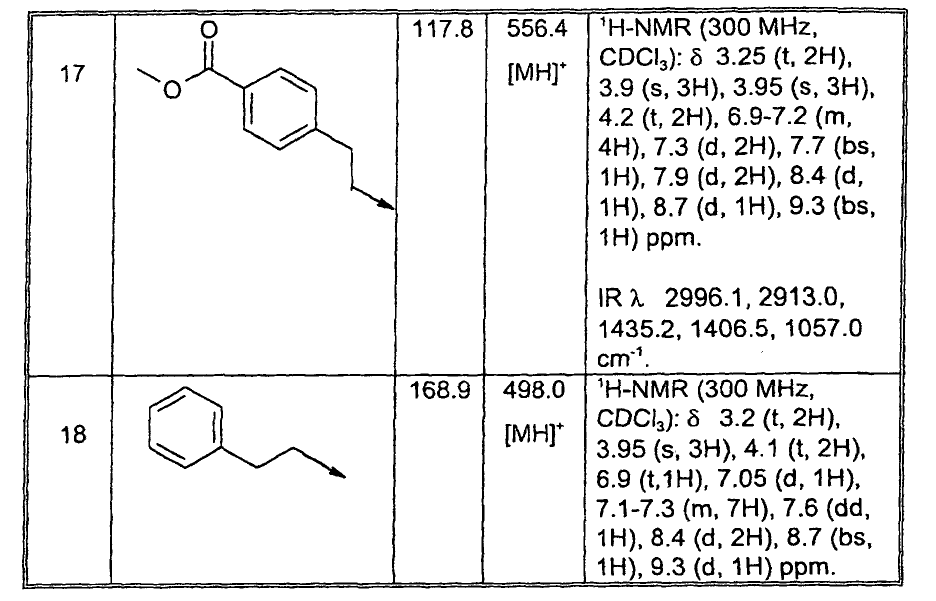

### EXAMPLE1

### N-[ 6-Methoxy-5-(2-methoxyphenoxy)-2-(3-pyridazinyl)-4-pyrimidinyl]-2-(1-naphthyl)ethanesulfonamide

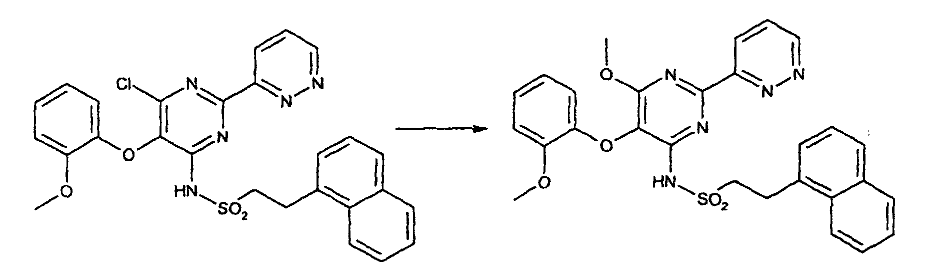

To a solution of *N*-[ 6-chloro-5-(2-methoxyphenoxy)-2-(3-pyridazinyl)-4-pyrimidinyl]-2-(1-naphthyl)ethanesulfonamide (0.088g, 0.17mmol)(Preparation 14) in methanol (5ml) was added sodium methoxide (25%w/v in methanol, 1.0 ml). The mixture was stirred vigorously for 72 hours at 60°C. and then quenched with aqueous hydrochloric acid (2M, 1ml). Ice-cold water (2ml) was added and a white precipitate formed. The resultant mixture was partitioned between water (5 ml) and dichloromethane (3x10ml). The combined organic extracts were washed with water (3x10ml), dried (magnesium sulfate) and concentrated under vacuum to give the title compound as a yellow solid (95% pure by HPLC). (0.065g).
¹H-NMR (300 MHz, CDCl₃) δ 3.55 (t, 2H), 3.9 (s, 3H), 4.05 (t, 2H), 4.12 (s, 3H), 6.85 (t, 1H), 6.9 (d, 1 H), 7.1 (m, 2H), 7.2 (m, 1 H), 7.3 (m, 3H), 7.4 (m, 1H), 7.65 (m, 1H), 7.75 (d, 1H), 7.85 (d, 1H), 8.25 (d, 1 H), 9.2 (d, 1H) ppm.
LRMS (-ve ion) found: m/z 542 (M-H). C₂₈H₂₅N₅O₅S-H requires m/z 542
IR (NaCI disk) λ 2360.6, 2253.7, 1584.9, 1498.6, 1405.6, 1328.9, 1096.2, 907.1 cm⁻¹

The compounds of the following tabulated Examples of the general formula: 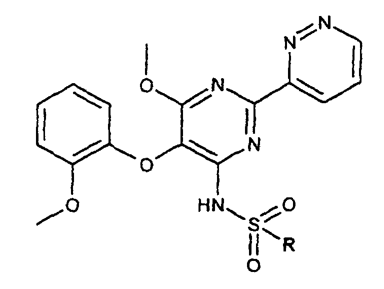 were prepared by a similar method to that of Example 1 using the appropriate 6-chloropyrimidines of Preparations 15-18.

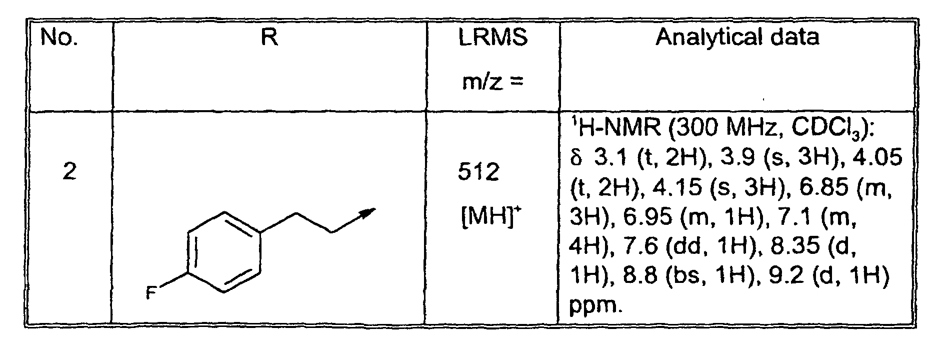

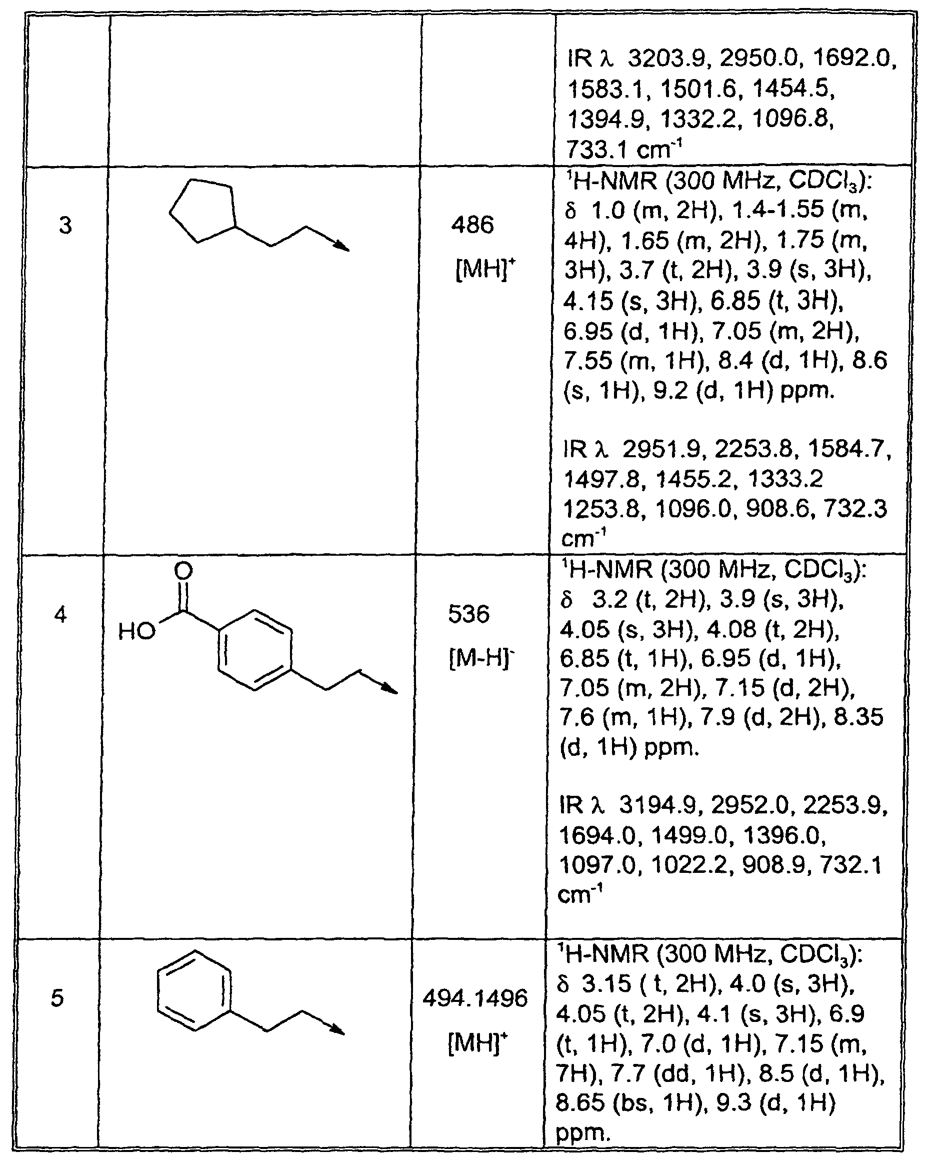

## Claims

1. A compound of formula (I): 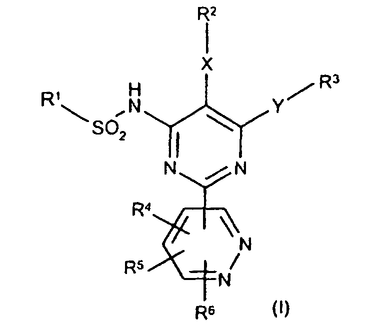 wherein:
R¹ is selected from
(CR⁷R⁸)ₙ-(C₃₋₈ cycloalkyl), (CR⁷R⁸)ₙ-het¹, (CR⁷R⁸)ₙ-aryl¹, NR⁹R¹⁰ and (CR⁷R⁸)ₙ-het², where R⁷ and R⁸ are independently selected from H and C₁₋₆ alkyl, and where
R⁹ and R¹⁰ are independently selected from H or C₁₋₆ alkyl (optionally substituted by one of aryl¹, het¹, het² or (C₃₋₈ cycloalkyl));
n is 2-6;
R² is
a) phenyl, optionally fused with het¹ or het²,
b) naphthyl, optionally fused with het¹ or het², or
c) het²,
said groups (a), (b) and (c) optionally substituted by C₁₋₆ alkyl, O(C₁₋₆ alkyl), halo, het³, het⁴, aryl², CO₂R¹¹, OC(O)R¹¹, CONR¹¹R¹², or S(O)ₚR¹¹ ;
R³ is
e) C₁₋₆ alkyl,
f) C₂₋₆ alkenyl,
g) C₂₋₆ alkynyl,
h) C₃₋₈ cycloalkyl
Where groups (e), (f), (g) and (h) may be optionally substituted by
OR¹¹, halo, NHC(O)C₁₋₆ alkyl, Ohet¹, Ohet², OC(O)NHhet¹, OC(O)NHhet², NH₂, NHC(O)Ohet² or NHC(O)NHhet²;
X is O, S(O)ₚ, NH or a direct link;
Y is O, S(O)ₚ, or NH;
p is 0,1 or 2;
R⁴, R⁵ and R⁶ are each independently selected from
H, halo, S(O)ₚR⁹, het¹, het², aryl¹, O(C₁₋₆ alkyl) and C₁₋₆ alkyl (optionally substituted by halo, OR⁹ or NH₂);
R¹¹ and R¹² are independently selected from H and C₁₋₆ alkyl;
wherein "aryl¹" is a C₆₋₁₄ aromatic carbocycle, including mono-, bi- and tri-cyclic systems;
"aryl²" is a C₆₋₁₀ aromatic carbocycle, including mono- and bi-cyclic systems;
"het¹" is a 3-8 membered ring containing 1-3 heteroatoms, each independently selected from N, O and S, said ring being saturated or partially unsaturated and optionally benzofused;
"het²" is a 5 or 6 membered aromatic ring containing 1-3 heteroatoms, each independently selected from N, O and S, said ring being optionally benzofused;
"het³" is a 3-6 membered ring containing 1-3 heteroatoms, each independently selected from N, O and S, said ring being saturated or partially unsaturated and optionally benzofused;
"het⁴" is a 5 or 6 membered aromatic ring containing 1-3 heteroatoms, each independently selected from N, O and S, said ring being optionally benzofused;
and wherein
"het¹", "het²", and "aryl¹" may optionally be substituted by 1 to 3 substituents independently selected from
halo, CO₂R⁹, OCOR⁹, OH, het⁴, S(O)ₚR¹¹, O(C₁₋₆ alkyl) or C₁₋₆ alkyl (optionally substituted by OH, O(C₁₋₆ alkyl) or halo);
a 6-14 membered aromatic carbocycle includes phenyl, naphthyl, indenyl, anthryl and phenanthryl;
and the pharmaceutically acceptable salts, solvates, and polymorphs thereof.

2. A compound, salt, or solvate according to claim 1 wherein the pyrimidine ring is attached at the 3 or 4 position of the pyridazine ring.

3. A compound, salt, or solvate according to any previous claim wherein R¹ is (CR⁷R⁸)ₙ-aryl¹, (CR⁷R⁸)ₙ-het², or (CR⁷R⁸)ₙ-(C₃₋₈ cycloalkyl) and where n is 2, 3, 4, 5 or 6.

4. A compound, salt, or solvate according to any previous claim wherein R¹ is (CR⁷R⁸)ₙ-aryl¹ or (CR⁷R⁸)ₙ-(C₃₋₈ cycloalkyl) and where n is 2 or 3.

5. A compound, salt, or solvate according to any previous claim wherein R¹ is (CR⁷R⁸)ₙ-aryl¹ or (CR⁷R⁸)ₙ-(C₃₋₈ cycloalkyl) and where n is 2.

6. A compound, salt, or solvate according to any previous claim wherein R² is phenyl or het², both optionally substituted by C₁₋₆ alkyl, O(C₁₋₆ alkyl), halo, het³, het⁴, aryl², CO₂R¹¹, OC(O)R¹¹ or CONR¹¹R¹².

7. A compound, salt, or solvate according to any previous claim wherein R² is phenyl optionally substituted by C₁₋₆ alkyl, O(C₁₋₆ alkyl), halo, het³, het⁴, aryl², CO₂R¹¹, OC(O)R¹¹or CONR¹¹R¹².

8. A compound, salt, or solvate according to any previous claim wherein R² is phenyl optionally substituted by C₁₋₃ alkyl, O(C₁₋₃ alkyl), F or Cl.

9. A compound, salt, or solvate according to any previous claim wherein R³ is C₁₋₆ alkyl, C₂₋₆ alkynyl or C₃₋₈ cycloalkyl, said alkyl, alkynyl and cycloalkyl groups optionally substituted by
OR¹¹, halo, NHC(O)C₁₋₆ alkyl, Ohet¹, Ohet², OC(O)NHhet¹ or OC(O)NHhet².

10. A compound, salt, or solvate according to any previous claim wherein R³ is C₂₋₃ alkynyl, or C₁₋₃ alkyl (optionally substituted by
OR¹¹, halo, NHC(O)C₁₋₆ alkyl, Ohet¹, Ohet², OC(O)NHhet¹ or OC(O)NHhet²).

11. A compound, salt, or solvate according to any previous claim wherein R³ is C₁₋₃ alkyl, CH₂CH₂OH, CH₂C≡CH, CH₂CH₂F or CH₂CH₂OCH₃.

12. A compound, salt, or solvate according to any previous claim wherein R⁴, R⁵ and R⁶ are selected from H, O(C₁₋₆ alkyl), S(C₁₋₆ alkyl), phenyl, halo, het¹, het² and C₁₋₆ alkyl (optionally substituted by halo or NH₂).

13. A compound, salt, or solvate according to any previous claim wherein R⁴ and R⁵ are hydrogen and R⁶ is selected from H, O(C₁₋₆ alkyl) and C₁₋₆ alkyl (optionally substituted by halo).

14. A compound, salt, or solvate according to any previous claim wherein R⁴ and R⁵ are hydrogen and R⁶ is selected from H or C₁₋₆ alkyl.

15. A compound, salt, or solvate according to any previous claim wherein R⁷ and R⁸ are independently selected from H and C₁₋₃ alkyl.

16. A compound, salt, or solvate according to any previous claim wherein R⁷ and R⁸ are independently selected from H and CH₃.

17. A compound, salt, or solvate according to any previous claim wherein R⁷ and R⁸ are H.

18. A compound, salt, or solvate according to any previous claim wherein R⁹ and R¹⁰ are selected from H and C₁₋₃ alkyl (optionally substituted by aryl¹, het¹, het² and C₃₋₈ cycloalkyl).

19. A compound, salt, or solvate according to any previous claim wherein R⁹ and R¹⁰ are selected from H and C₁₋₃ alkyl (optionally substituted by aryl¹ or het¹).

20. A compound, salt, or solvate according to any previous claim wherein R⁹ and R¹⁰ are H.

21. A compound, salt, or solvate according to any previous claim wherein R¹¹ and R¹² are selected from H and C₁₋₃ alkyl.

22. A compound, salt, or solvate according to any previous claim wherein R¹¹ and R¹² are selected from H and CH₃.

23. A compound, salt, or solvate according to any previous claim wherein X is O, NH or a direct link.

24. A compound, salt, or solvate according to any previous claim wherein X is O.

25. A compound, salt, or solvate according to any previous claim wherein Y is O or NH.

26. A compound, salt, or solvate according to any previous claim wherein Y is O.

27. A compound, salt, or solvate according to any previous claim wherein n is 2, 3 or 4.

28. A compound, salt, or solvate according to any previous claim wherein n is 2.

29. A compound according to claim 1 which is selected from:
*N*-[ 6-Methoxy-5-(2-methoxyphenoxy)-2-(3-pyridazinyl)-4-pyrimidinyl]-2-(1-naphthyl)ethanesulfonamide,
*N*-[ 6-Methoxy-5-(2-methoxyphenoxy)-2-(3-pyridazinyl)-4-pyrimidinyl]-2-(4-fluorophenyl)ethanesulfonamide,
*N*-[ 6-Methoxy-5-(2-methoxyphenoxy)-2-(3-pyridazinyl)-4-pyrimidinyl]-2-cyclopentylethanesulphonamide,
*N*-[ 6-Methoxy-5-(2-methoxyphenoxy)-2-(3-pyridazinyl)-4-pyrimidinyl]-2-(4-carboxyphenyl)ethanesulfonamide,
*N*-[ 6-Methoxy-5-(2-methoxyphenoxy)-2-(3-pyridazinyl)-4-pyrimidinyl]-2-phenylethanesulphonamide
and the salts and solvates thereof.

30. A pharmaceutical composition comprising a substance according to any one of claims 1 to 29, and a pharmaceutically acceptable diluent, carrier or adjuvant.

31. A substance according to any one of claims 1 to 29 for use in medicine.

32. The use of a substance according to any one of claims 1 to 29 in the manufacture of a medicament for the treatment of an endothelin mediated condition.

33. The use of a compound of claims 1 to 30 for the manufacture of a medicament for the treatment of a condition mediated by endothelin comprising administration of a therapeutically-effective amount of a substance according to any one of claims 1 to 29.

34. A substance according to any one of claims 1 to 29 for use in the treatment of an endothelin mediated condition.

35. A process for making a compound of formula (I) as defined in claim 1 which comprises:
a. condensation of compounds of formula (V) and (VI), where R^{10a} is a C₁₋₆ alkyl or phenyl group, to make a compound of formula (IV): 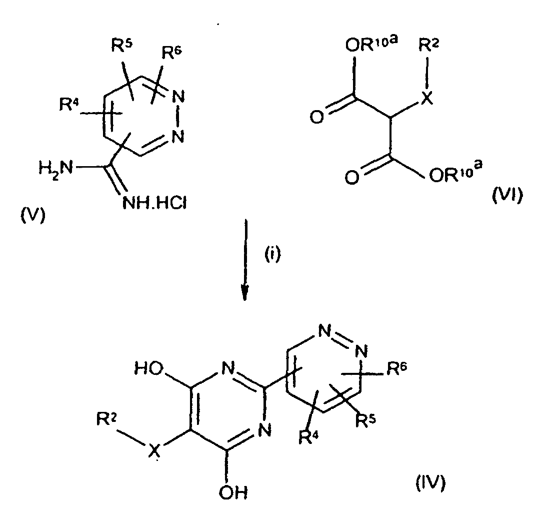
b. chlorination of a compound of formula (IV), in the presence of a base, and where required with an inert solvent, to make a compound of formula (III): 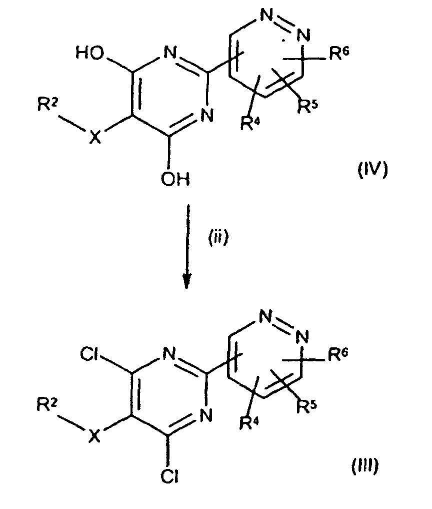
c. reaction of a compound of formula (III) with a compound of formula R¹SO₂NH₂, in the presence of a base, heated, to make a compound of formula (II) 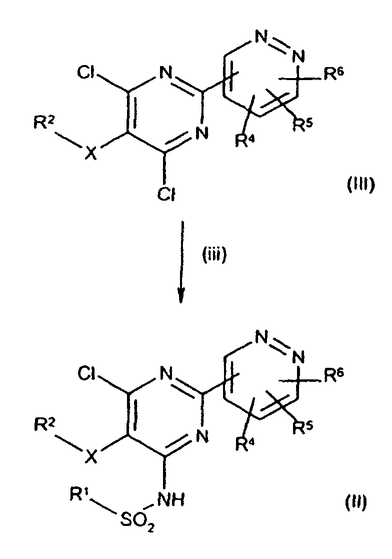
d. reaction of a compound of formula (II) with a compound of formula R³YH, in the presence of a base, to make a compound of formula (I).

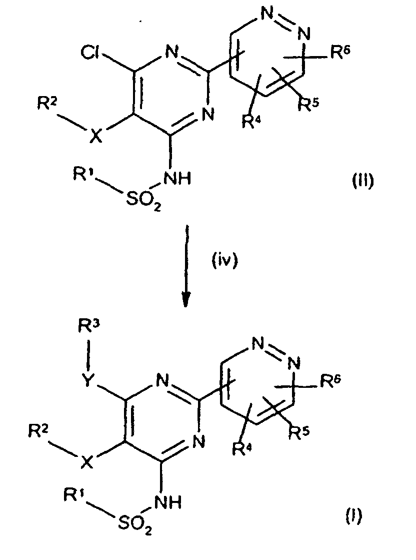

36. A compound of formula (IV) as defined in claim 35.

37. A compound of formula (III) as defined in claim 35.

38. A compound of formula (II) as defined in claim 35.

39. A compound of formula (V) as defined in claim 35.

## Patentansprüche

1. Verbindung der Formel (I): 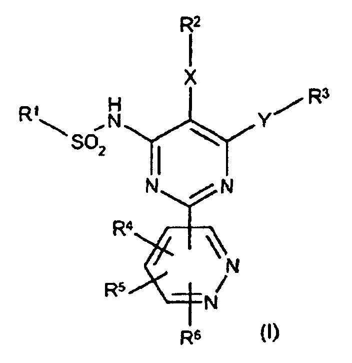 worin:
R¹ ausgewählt ist aus
(CR⁷R⁸)ₙ- (C₃₋₈-Cycloalkyl), (CR⁷R⁸)ₙ-Het¹, (CR⁷R⁸)ₙ-Aryl¹,
NR⁹R¹⁰ und (CR⁷R⁸)ₙ-Het², worin R⁷ und R⁸ unabhängig ausgewählt sind aus H und C₁₋₆-Alkyl und
worin
R⁹ und R¹⁰ unabhängig ausgewählt sind aus H oder C₁₋₆-Alkyl (gegebenenfalls substituiert mit einem von Aryl¹, Het¹, Het² oder (C₃₋₈-Cycloalkyl));
n 2-6 ist;
R² bedeutet
a) Phenyl, gegebenenfalls kondensiert mit Het¹ oder Het²,
b) Naphthyl, gegebenenfalls kondensiert mit Het¹ oder Het², oder
c) Het²,
wobei die Gruppen (a), (b) und (c) gegebenenfalls mit C₁₋₆-Alkyl, O(C₁₋₆-Alkyl), Halogen, Het³, Het⁴, Aryl², CO₂R¹¹, OC (O) R¹¹, CONR¹¹R¹² oder S(O)ₚR¹¹ substituiert sind; R³ bedeutet
e) C₁₋₆-Alkyl,
f) C₂₋₆-Alkenyl,
g) C₂₋₆-Alkinyl,
h) C₃₋₈-Cycloalkyl,
worin Gruppen (e), (f), (g) und (h) gegebenenfalls substituiert sein können mit
OR¹¹, Halogen, NHC(O)C₁₋₆-Alkyl, OHet¹, OHet², OC(O)NHHet¹, OC(O)NHHet², NH₂, NHC(O)OHet² oder NHC(O)NHHet²;
X O, S(O)ₚ, NH oder eine direkte Bindung darstellt;
Y O, S(O)ₚ oder NH darstellt;
p 0, 1 oder 2 ist;
R⁴, R⁵ und R⁶ jeweils unabhängig ausgewählt sind aus
H, Halogen, S(O)ₚR⁹, Het¹, Het², Aryl¹, O(C₁₋₆-Alkyl) und C₁₋₆-Alkyl (gegebenenfalls substituiert mit Halogen, OR⁹ oder NH₂);
R¹¹ und R¹² unabhängig ausgewählt sind aus H und C₁₋₆-Alkyl;
worin "Aryl¹" einen aromatischen C₆₋₁₄-Carbocyclus, einschließlich mono-, bi- und tricyclischer Systeme, darstellt;
"Aryl²" einen aromatischen C₆₋₁₀-Carbocyclus, einschließlich mono- und bicyclischer Systeme, darstellt;
"Het¹" einen 3-8-gliedrigen Ring, der 1-3 Heteroatome enthält, jeweils unabhängig ausgewählt aus N, O und S, darstellt, wobei der Ring gesättigt oder teilweise ungesättigt und gegebenenfalls benzokondensiert ist;
"Het²" einen 5 oder 6-gliedrigen aromatischen Ring, der 1-3 Heteroatome enthält, jeweils unabhängig ausgewählt aus N, O und S, darstellt, wobei der Ring gegebenenfalls benzokondensiert ist;
"Het³" einen 3-6-gliedrigen Ring, der 1-3 Heteroatome enthält, jeweils unabhängig ausgewählt aus N, O und S, darstellt, wobei der Ring gesättigt oder teilweise ungesättigt und gegebenenfalls benzokondensiert ist;
"Het⁴" einen 5 oder 6-gliedrigen aromatischen Ring, der 1-3 Heteroatome enthält, jeweils unabhängig ausgewählt aus N, O und S, darstellt, wobei der Ring gegebenenfalls benzokondensiert ist;
und worin
"Het¹", "Het²" und "Aryl¹" gegebenenfalls mit 1 bis 3 Substituenten, unabhängig ausgewählt aus
Halogen, CO₂R⁹, OCOR⁹, OH, Het⁴, S(O)ₚR¹¹, O(C₁₋₆-Alkyl) oder C₁₋₆-Alkyl (gegebenenfalls substituiert mit OH, O(C₁₋₆-Alkyl) oder Halogen), substituiert sein können;
ein 6-14-gliedriger aromatischer Carbocyclus Phenyl, Naphthyl, Indenyl, Anthryl und Phenanthryl einschließt;
und die pharmazeutisch verträglichen Salze, Solvate und Polymorphe davon.

2. Verbindung, Salz oder Solvat nach Anspruch 1, worin der Pyrimidinring an die Position 3 oder 4 des Pyridazinrings gebunden ist.

3. Verbindung, Salz oder Solvat nach einem beliebigen vorangehenden Anspruch, worin R¹ (CR⁷R⁸)ₙ-Aryl¹, (CR⁷R⁸)ₙ-Het² oder (CR⁷R⁸)ₙ-(C₃₋₈-Cycloalkyl) darstellt und worin n 2, 3, 4, 5 oder 6 ist.

4. Verbindung, Salz oder Solvat nach einem beliebigen vorangehenden Anspruch, worin R¹ (CR⁷R⁸)ₙ-Aryl¹ oder (CR⁷R⁸)ₙ-(C₃₋₈-Cycloalkyl) darstellt und worin n 2 oder 3 ist.

5. Verbindung, Salz oder Solvat nach einem beliebigen vorangehenden Anspruch, worin R¹ (CR⁷R⁸)ₙ-Aryl¹ oder (CR⁷R⁸)ₙ-(C₃₋₈-Cycloalkyl) darstellt und worin n 2 ist.

6. Verbindung, Salz oder Solvat nach einem beliebigen vorangehenden Anspruch, worin R² Phenyl oder Het², beide gegebenenfalls substituiert mit C₁₋₆-Alkyl, O(C₁₋₆-Alkyl), Halogen, Het³, Het⁴, Aryl², CO₂R¹¹, OC(O)R¹¹ oder CONR¹¹R¹², darstellt.

7. Verbindung, Salz oder Solvat nach einem beliebigen vorangehenden Anspruch, worin R² Phenyl, gegebenenfalls substituiert mit C₁₋₆-Alkyl, O(C₁₋₆-Alkyl), Halogen, Het³, Het⁴, Aryl², CO₂R¹¹, OC(O)R¹¹ oder CONR¹¹R¹², darstellt.

8. Verbindung, Salz oder Solvat nach einem beliebigen vorangehenden Anspruch, worin R² Phenyl, gegebenenfalls substituiert mit C₁₋₃-Alkyl, O(C₁₋₃-Alkyl), F oder Cl, darstellt.

9. Verbindung, Salz oder Solvat nach einem beliebigen vorangehenden Anspruch, worin R³ C₁₋₆-Alkyl, C₂₋₆-Alkinyl oder C₃₋₆-Cycloalkyl darstellt, wobei die Alkyl-, Alkinyl- und Cycloalkylgruppen gegebenenfalls mit OR¹¹, Halogen, NHC(O)C₁₋₆-Alkyl, OHet¹, OHet², OC(O)NHHet¹ oder OC(O)NHHet² substituiert sind.

10. Verbindung, Salz oder Solvat nach einem beliebigen vorangehenden Anspruch, worin R³ C₂₋₃-Alkinyl oder C₁₋₃-Alkyl (gegebenenfalls substituiert mit OR¹¹, Halogen, NHC(O)C₁₋₆-Alkyl, OHet¹, OHet², OC(O)NHHet¹ oder OC(O)NHHet²) darstellt.

11. Verbindung, Salz oder Solvat nach einem beliebigen vorangehenden Anspruch, worin R³ C₁₋₃-Alkyl, CH₂CH₂OH, CH₂C≡CH, CH₂CH₂F oder CH₂CH₂OCH₃ darstellt.

12. Verbindung, Salz oder Solvat nach einem beliebigen vorangehenden Anspruch, worin R⁴, R⁵ und R⁶ aus H, O(C₁₋₆-Alkyl), S(C₁₋₆-Alkyl), Phenyl, Halogen, Het¹, Het² und C₁₋₆-Alkyl (gegebenenfalls substituiert mit Halogen oder NH₂) ausgewählt sind.

13. Verbindung, Salz oder Solvat nach einem beliebigen vorangehenden Anspruch, worin R⁴ und R⁵ Wasserstoff darstellen, und R⁶ aus H, O(C₁₋₆-Alkyl) und C₁₋₆-Alkyl (gegebenenfalls substituiert mit Halogen) ausgewählt ist.

14. Verbindung, Salz oder Solvat nach einem beliebigen vorangehenden Anspruch, worin R⁴ und R⁵ Wasserstoff darstellen, und R⁶ aus H oder C₁₋₆-Alkyl ausgewählt ist.

15. Verbindung, Salz oder Solvat nach einem beliebigen vorangehenden Anspruch, worin R⁷ und R⁸ unabhängig aus H oder C₁₋₃-Alkyl ausgewählt sind.

16. Verbindung, Salz oder Solvat nach einem beliebigen vorangehenden Anspruch, worin R⁷ und R⁸ unabhängig aus H oder CH₃ ausgewählt sind.

17. Verbindung, Salz oder Solvat nach einem beliebigen vorangehenden Anspruch, worin R⁷ und R⁸ H darstellen.

18. Verbindung, Salz oder Solvat nach einem beliebigen vorangehenden Anspruch, worin R⁹ und R¹⁰ aus H oder C₁₋₃-Alkyl (gegebenenfalls substituiert mit Aryl¹, Het¹, Het² und C₃₋₈-Cyclalkyl) ausgewählt sind.

19. Verbindung, Salz oder Solvat nach einem beliebigen vorangehenden Anspruch, worin R⁹ und R¹⁰ aus H oder C₁₋₃-Alkyl (gegebenenfalls substituiert mit Aryl¹ oder Het¹) ausgewählt sind.

20. Verbindung, Salz oder Solvat nach einem beliebigen vorangehenden Anspruch, worin R⁹ und R¹⁰ H darstellen.

21. Verbindung, Salz oder Solvat nach einem beliebigen vorangehenden Anspruch, worin R¹¹ und R¹² aus H und C₁₋₃-Alkyl ausgewählt sind.

22. Verbindung, Salz oder Solvat nach einem beliebigen vorangehenden Anspruch, worin R¹¹ und R¹² aus H und CH₃ ausgewählt sind.

23. Verbindung, Salz oder Solvat nach einem beliebigen vorangehenden Anspruch, worin X O, NH oder eine direkte Bindung darstellt.

24. Verbindung, Salz oder Solvat nach einem beliebigen vorangehenden Anspruch, worin X O darstellt.

25. Verbindung, Salz oder Solvat nach einem beliebigen vorangehenden Anspruch, worin Y O oder NH darstellt.

26. Verbindung, Salz oder Solvat nach einem beliebigen vorangehenden Anspruch, worin Y 0 darstellt.

27. Verbindung, Salz oder Solvat nach einem beliebigen vorangehenden Anspruch, worin n 2, 3 oder 4 ist.

28. Verbindung, Salz oder Solvat nach einem beliebigen vorangehenden Anspruch, worin n 2 ist.

29. Verbindung nach Anspruch 1, die ausgewählt ist aus:
N-[6-Methoxy-5-(2-methoxyphenoxy)-2-(3-pyridazinyl)-4-pyrimidinyl]-2-(1-naphthyl)ethansulfonamid,
N-[6-Methoxy-5-(2-methoxyphenoxy)-2-(3-pyridazinyl)-4-pyrimidinyl]-2-(4-fluorphenyl)ethansulfonamid,
N-[6-Methoxy-5-(2-methoxyphenoxy)-2-(3-pyridazinyl)-4-pyrimidinyl]-2-cyclopentylethansulfonamid,
N-[6-Methoxy-5-(2-methoxyphenoxy)-2-(3-pyridazinyl)-4-pyrimidinyl]-2-(4-carboxyphenyl)ethansulfonamid,
N-[6-Methoxy-5-(2-methoxyphenoxy)-2-(3-pyridazinyl)-4-pyrimidinyl]-2-phenylethansulfonamid
und die Salze und Solvate davon.

30. Pharmazeutische Zusammensetzung, umfassend eine Substanz nach einem beliebigen der Ansprüche 1 bis 29 und ein pharmazeutisch verträgliches Verdünnungsmittel, Träger- oder Hilfsmittel.

31. Substanz nach einem beliebigen der Ansprüche 1 bis 29 zur Verwendung in der Medizin.

32. Verwendung einer Substanz nach einem beliebigen der Ansprüche 1 bis 29 bei der Herstellung eines Arzneimittels für die Behandlung eines durch Endothelin vermittelten Zustands.

33. Verwendung einer Verbindung nach Ansprüchen 1 bis 30 zur Herstellung eines Arzneimittels für die Behandlung eines durch Endothelin vermittelten Zustands, umfassend Verabreichung einer therapeutisch wirksamen Menge einer Substanz nach einem der Ansprüche 1 bis 29.

34. Substanz nach einem der Ansprüche 1 bis 29 zur Verwendung bei der Behandlung eines Endothelin vermittelten Zustands.

35. Verfahren zur Herstellung einer Verbindung der Formel (I) wie in Anspruch 1 definiert, das umfasst:
a. Kondensation von Verbindungen der Formel (V) und (VI), worin R^{10a} eine C₁₋₆-Alkyl- oder Phenylgruppe darstellt, zur Herstellung einer Verbindung der Formel (IV): 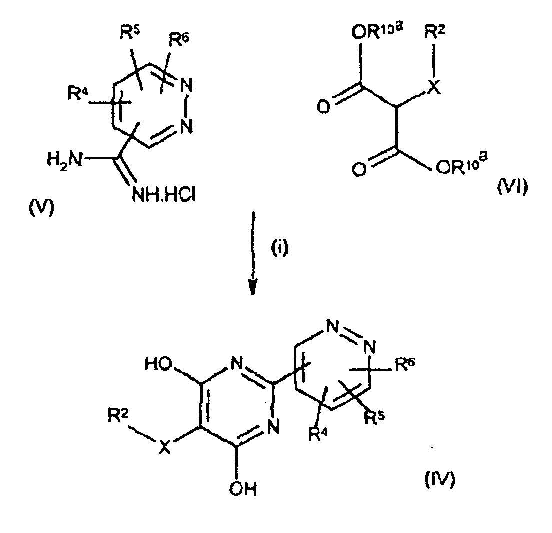
b. Chlorierung einer Verbindung der Formel (IV) in Gegenwart einer Base und, falls erforderlich, mit einem inerten Lösungsmittel zur Herstellung einer Verbindung der Formel (III): 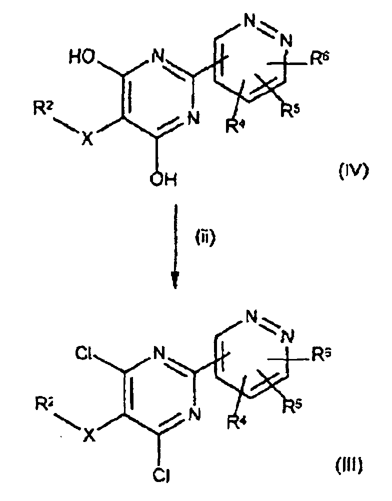
c. Reaktion einer Verbindung der Formel (III) mit einer Verbindung der Formel R¹SO₂NH₂ in Gegenwart einer Base, Erhitzen, zur Herstellung einer Verbindung der Formel (II): 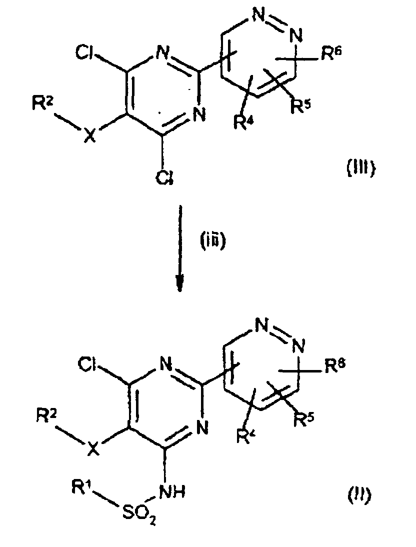 oder
d. Reaktion einer Verbindung der Formel (II) mit einer Verbindung der Formel R³YH in Gegenwart einer Base, zur Herstellung einer Verbindung der Formel (I):

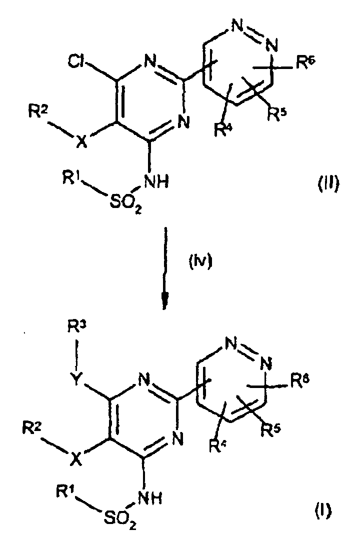

36. Verbindung der Formel (IV) wie in Anspruch 35 definiert.

37. Verbindung der Formel (III) wie in Anspruch 35 definiert.

38. Verbindung der Formel (II) wie in Anspruch 35 definiert.

39. Verbindung der Formel (V) wie in Anspruch 35 definiert.

## Revendications

1. Composé de formule (I) : 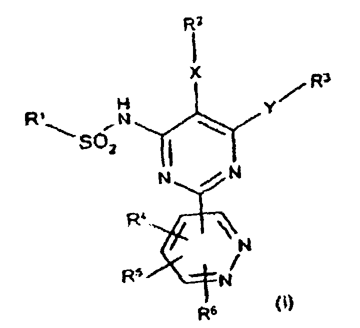 dans laquelle :
R¹ est choisi entre des groupes
(CR⁷R⁸)ₙ-(cycloalkyle en C₃ à C₈), (CR⁷R⁸)ₙ-het¹, (CR⁷R⁸)ₙ-aryle¹, NR⁹R¹⁰ et (CR⁷R⁸)ₙ-het², dans lesquels R⁷ et R⁸ sont choisis indépendamment entre H et un groupe alkyle en C₁ à C₆, et
R⁹ et R¹⁰ sont choisis indépendamment entre H et un groupe alkyle en C₁ à C₆ (facultativement substitué avec un substituant aryle¹, het¹, het² ou cycloalkyle en C₃ à C₈) ;
n a une valeur de 2 à 6 ;
R² représente un groupe
a) phényle, facultativement condensé avec un groupe het¹ ou het² ;
b) un groupe naphtyle, facultativement condensé avec un groupe het¹ ou het², ou
c) un groupe het²,
lesdits groupes (a), (b) et (c) étant facultativement substitués avec des substituants alkyle en C₁ à C₆, O(alkyle en C₁ à C₆), halogéno, het³, het⁴, aryle², CO₂R¹¹, OC(O)R¹¹, CONR¹¹R¹², ou S(O)ₚR¹¹,
R³ représente un groupe
e) alkyle en C₁ à C₆,
f) alcényle en C₂ à C₆,
g) alcynyle en C₂ à C₆,
h) cycloalkyle en C₃ à C₈
les groupes (e), (f), (g) et (h) pouvant être facultativement substitués avec des substituants
OR¹¹, halogéno, NHC(O)(alkyle en C₁ à C₆), Ohet¹, Ohet², OC(O)NHhet¹, OC(O)NHhet², NH₂, NHC(O)Ohet² ou NHC(O)NHhet² ;
X représente un atome de O, un groupe S(O)p, un groupe NH ou une liaison directe ;
Y représente un atome de 0, un groupe S(O)ₚ, ou un groupe NH ;
p est égal à 0 ou 2 ;
R⁴, R⁵ et R⁶ sont choisis chacun indépendamment entre H, des groupes halogéno, S(O)ₚR⁹, het¹, het², aryle¹, O(alkyle en C₁ à C₆) et alkyle en C₁ à C₆ (facultativement substitué avec un substituant halogéno, OR⁹ ou NH₂) ;
R¹¹ et R¹² sont choisis indépendamment entre H et un groupe alkyle en C₁ à C₆ ;
où le groupe "aryle¹" est un carbocycle aromatique en C₆ à C₁₄, y compris des systèmes mono-, bi- et tricycliques ;
le groupe "aryle²" est un carbocycle aromatique en C₆ à C₁₀, y compris des systèmes mono-, bi- et tricycliques ;
le groupe "het¹" est un noyau tri- à octogonal contenant 1 à 3 hétéroatomes, chacun choisi indépendamment entre N, O et S, ledit noyau étant saturé ou partiellement insaturé et facultativement benzocondensé ;
le groupe "het²" est un noyau aromatique penta- ou hexagonal contenant 1 à 3 hétéroatomes, chacun choisi indépendamment entre N, O et S, ledit noyau étant facultativement benzocondensé ;
le groupe "het³" est un noyau tri- à hexagonal contenant 1 à 3 hétéroatomes, chacun choisi indépendamment entre N, O et S, ledit noyau étant saturé ou partiellement insaturé et facultativement benzocondensé ;
le groupe "het⁴" est noyau aromatique penta- ou hexagonal contenant 1 à 3 hétéroatomes, chacun choisi indépendamment entre N, O et S, ledit noyau étant facultativement benzocondensé ;
et où
les groupes "het¹", "het²", "aryle¹" peuvent être facultativement substitués avec 1 à 3 substituants choisis indépendamment entre des substituants
halogéno, CO₂R⁹, OCOR⁹, OH, het⁴, S(O)ₚR¹¹, O(alkyle en C₁ à C₆) et alkyle en C₁ à C₆ (facultativement substitué avec un substituant OH, O(alkyle en C₁ à C₆) ou halogéno) ;
un carbocycle aromatique de 6 à 14 membres comprend les groupes phényle, naphtyle, indényle, anthryle et phénanthryle ;
et ses sels, produits de solvatation et formes polymorphes pharmaceutiquement acceptables.

2. Composé, sel ou produit de solvatation suivant la revendication 1, dans lequel le noyau pyrimidine est fixé en position 3 ou 4 du noyau pyridazine.

3. Composé, sel ou produit de solvatation suivant l'une quelconque des revendications précédentes, dans lequel R¹ représente un groupe (CR⁷R⁸)ₙ-aryle¹, (CR⁷R⁸)ₙ-het², ou (CR⁷R⁸)ₙ-(cycloalkyle en C₃ à C₈) dans lequel n est égal à 2, 3, 4, 5 ou 6.

4. Composé, sel ou produit de solvatation suivant l'une quelconque des revendications précédentes, dans lequel R¹ représente un groupe (CR⁷R⁸)ₙ-aryle¹ ou (CR⁷R⁸)ₙ-(cycloalkyle en C₃ à C₈) dans lequel n est égal à 2 ou 3.

5. Composé, sel ou produit de solvatation suivant l'une quelconque des revendications précédentes, dans lequel R¹ représente un groupe (CR⁷R⁸)ₙ-aryle¹ ou (CR⁷R⁸)ₙ-(cyclo-alkyle en C₃ à C₈) dans lequel n est égal à 2.

6. Composé, sel ou produit de solvatation suivant l'une quelconque des revendications précédentes, dans lequel R² représente un groupe phényle ou het², ces deux groupes étant facultativement substitués avec un substituant alkyle en C₁ à C₆, O(alkyle en C₁ à C₆), halogéno, het³, het⁴, aryle², CO₂R¹¹, OC(O)R¹¹ ou CONR¹¹R¹².

7. Composé, sel ou produit de solvatation suivant l'une quelconque des revendications précédentes, dans lequel R² représente un groupe phényle facultativement substitué avec un substituant alkyle en C₁ à C₈, O(alkyle en C₁ à C₆), halogéno, het³, het⁴, aryle², CO₂R¹¹, OC(O)R¹¹ ou CONR¹¹R¹².

8. Composé, sel ou produit de solvatation suivant l'une quelconque des revendications précédentes, dans lequel R² représente un groupe phényle facultativement substitué avec un substituant alkyle en C₁ à C₃, O(alkyle en C₁ à C₃), F ou Cl.

9. Composé, sel ou produit de solvatation suivant l'une quelconque des revendications précédentes, dans lequel R³ représente un groupe alkyle en C₁ à C₆, alcynyle en C₂ à C₆ ou cycloalkyle en C₃ à C₈, lesdits groupes alkyle, alcynyle et cycloalkyle étant facultativement substitués avec des substituants OR¹¹, halogéno, NHC(O)(alkyle en C₁ à C₆), Ohet¹, Ohet², OC(O)NHhet¹ ou OC(O)NHhet².

10. Composé, sel ou produit de solvatation suivant l'une quelconque des revendications précédentes, dans lequel R³ représente un groupe alcynyle en C₂ ou C₃, ou alkyle en C₁ à C₃ (facultativement substitué avec un substituant OR¹¹, halogéno, NHC(O) (alkyle en C₁ à C₆), Ohet¹, Ohet², OC(O)NHhet¹ ou OC(O)NHhet²).

11. Composé, sel ou produit de solvatation suivant l'une quelconque des revendications précédentes, dans lequel R³ représente un groupe alkyle en C₁ à C₃, CH₂CH₂OH, CH₂C=CH, CH₂CH₂F ou CH₂CH₂OCH₃.

12. Composé, sel ou produit de solvatation suivant l'une quelconque des revendications précédentes, dans lequel R⁴, R⁵ et R⁶ sont choisis entre H, des groupes O(alkyle en C₁ à C₆), S (alkyle en C₁ à C₆), phényle, halogéno, het¹, het² et alkyle en C₁ à C₆ (facultativement substitué avec un substituant halogéno ou NH₂).

13. Composé, sel ou produit de solvatation suivant l'une quelconque des revendications précédentes, dans lequel R⁴ et R⁵ représentent des atomes d'hydrogène et R⁶ est choisi entre H, des groupes O(alkyle en C₁ à C₆) et alkyle en C₁ à C₆ (facultativement substitué avec un substituant halogéno).

14. Composé, sel ou produit de solvatation suivant l'une quelconque des revendications précédentes, dans lequel R⁴ et R⁵ représentent des atomes d'hydrogène et R⁶ est choisi entre H et un groupe alkyle en C₁ à C₆.

15. Composé, sel ou produit de solvatation suivant l'une quelconque des revendications précédentes, dans lequel R⁷ et R⁸ sont choisis indépendamment entre H et un groupe alkyle en C₁ à C₃.

16. Composé, sel ou produit de solvatation suivant l'une quelconque des revendications précédentes, dans lequel R⁷ et R⁸ sont choisis indépendamment entre H et un groupe CH₃.

17. Composé, sel ou produit de solvatation suivant l'une quelconque des revendications précédentes, dans lequel R⁷ et R⁸ représentent des atomes de H.

18. Composé, sel ou produit de solvatation suivant l'une quelconque des revendications précédentes, dans lequel R⁹ et R¹⁰ sont choisis entre H et un groupe alkyle en C₁ à C₃ (facultativement substitué avec un substituant aryle¹, het¹, het² ou cycloalkyle en C₃ à C₈).

19. Composé, sel ou produit de solvatation suivant l'une quelconque des revendications précédentes, dans lequel R⁹ et R¹⁰ sont choisis entre H et un groupe alkyle en C₁ à C₃ (facultativement substitué avec un substituant aryle¹ ou het¹).

20. Composé, sel ou produit de solvatation suivant l'une quelconque des revendications précédentes, dans lequel R⁹ et R¹⁰ représentent des atomes de H.

21. Composé, sel ou produit de solvatation suivant l'une quelconque des revendications précédentes, dans lequel R¹¹ et R¹² sont choisis entre H et un groupe alkyle en C₁ à C₃.

22. Composé, sel ou produit de solvatation suivant l'une quelconque des revendications précédentes, dans lequel R¹¹ et R¹² sont choisis entre H et un groupe CH₃.

23. Composé, sel ou produit de solvatation suivant l'une quelconque des revendications précédentes, dans lequel X représente un atome de O, un groupe NH ou une liaison directe.

24. Composé, sel ou produit de solvatation suivant l'une quelconque des revendications précédentes, dans lequel X représente un atome de O.

25. Composé, sel ou produit de solvatation suivant l'une quelconque des revendications précédentes, dans lequel Y représente un atome de O ou un groupe NH.

26. Composé, sel ou produit de solvatation suivant l'une quelconque des revendications précédentes, dans lequel y représente un atome de O.

27. Composé, sel ou produit de solvatation suivant l'une quelconque des revendications précédentes, dans lequel n est égal à 2, 3 ou 4.

28. Composé, sel ou produit de solvatation suivant l'une quelconque des revendications précédentes, dans lequel n est égal à 2.

29. Composé suivant la revendication 1, qui est choisi entre les suivants :
N-[6-méthoxy-5-(2-méthoxyphénoxy)-2-(3-pyridazinyl)-4-pyrimidinyl]-2-(1-naphthyl)éthanesulfonamide,
N-[6-méthoxy-5-(2-méthoxyphénoxy)-2-(3-pyridazinyl)-4-pyrimidinyl]-2-(4-fluorophényl)éthanesulfonamide,
N-[6-méthoxy-5-(2-méthoxyphénoxy)-2-(3-pyridazinyl)-4-pyrimidinyl]-2-cyclopentyléthanesulfonamide,
N-[6-méthoxy-5-(2-méthoxyphénoxy)-2-(3-pyridazinyl)-4-pyrimidinyl]-2-(4-carboxyphényl)éthanesulfonamide,
N-[6-méthoxy-5-(2-méthoxyphénoxy)-2-(3-pyridazinyl)-4-pyrimidinyl]-2-phényléthanesulfonamide,
et leurs sels et produits de solvatation.

30. Composition pharmaceutique comprenant une substance selon l'une quelconque des revendications 1 à 29 et un diluant, support ou adjuvant pharmaceutiquement acceptables.

31. Substance suivant l'une quelconque des revendications 1 à 29, destinée à être utilisée en médecine.

32. Utilisation d'une substance suivant l'une quelconque des revendications 1 à 29 dans la production d'un médicament destiné au traitement d'une affection à médiation par une endothéline.

33. Utilisation d'un composé suivant les revendications 1 à 30 pour la production d'un médicament destiné au traitement d'une affection à médiation par une endothéline, comprenant l'administration d'une quantité thérapeutiquement efficace d'une substance suivant l'une quelconque des revendications 1 à 29.

34. Substance suivant l'une quelconque des revendications 1 à 29, destinée à être utilisée dans le traitement d'une affection à médiation par une endothéline.

35. Procédé pour la préparation d'un composé de formule (I) tel que défini dans la revendication 1, qui comprend :
a. la condensation des composés de formule (V) et (VI) dans lesquelles R^{10a} représente un groupe alkyle en C₁ à C₆ ou un groupe phényle, pour préparer un composé de formule (IV) : 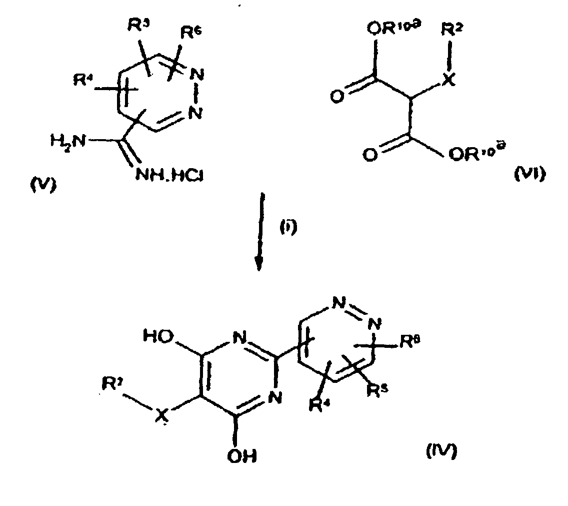
b. la chloration d'un composé de formule (IV), en présence d'une base et, lorsque cela est nécessaire, avec un solvant inerte, pour préparer un composé de formule (III) : 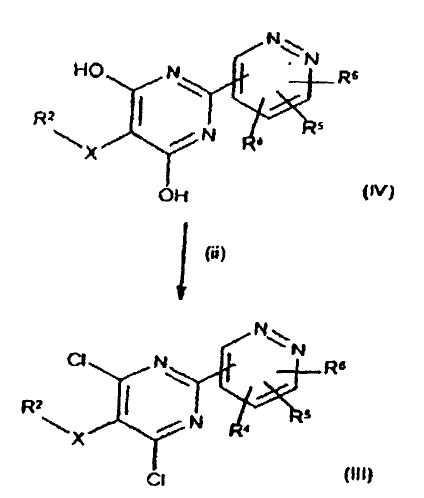
c. la réaction d'un composé de formule (III) avec un composé de formule R¹SO₂NH₂, en présence d'une base, à chaud, pour préparer un composé de formule (II) 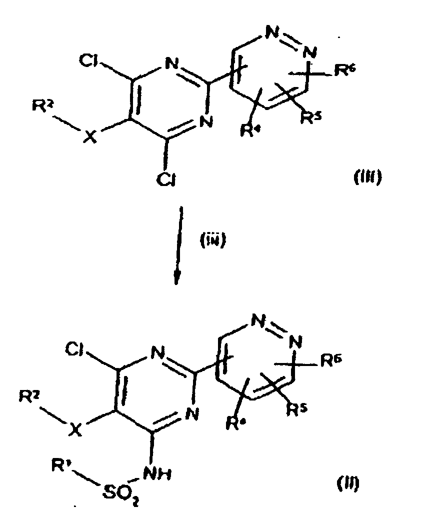
d. la réaction d'un composé de formule (II) avec un composé de formule R³YH, en présence d'une base, pour préparer un composé de formule (I).

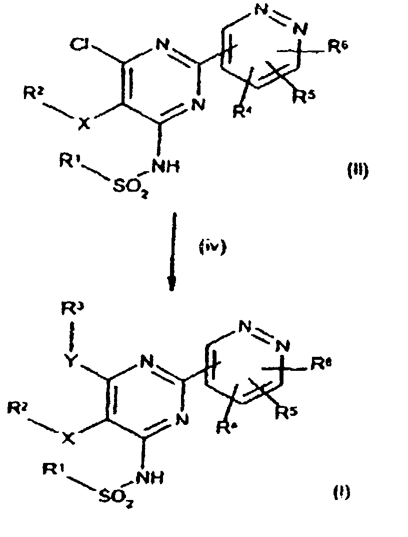

36. Composé de formule (IV) tel que défini dans la revendication 35.

37. Composé de formule (III) tel que défini dans la revendication 35.

38. Composé de formule (II) tel que défini dans la revendication 35.

39. Composé de formule (V) tel que défini dans la revendication 35.
